(19) Europäisches Patentamt
European Patent Office
Office européen des brevets

(11) **EP 1 782 780 A1**

(12) **EUROPEAN PATENT APPLICATION**
published in accordance with Art. 158(3) EPC

(43) Date of publication:
**09.05.2007 Bulletin 2007/19**

(51) Int Cl.:
**A61F 7/08** (2006.01)   **C09K 5/16** (2006.01)

(21) Application number: **05765809.8**

(22) Date of filing: **14.07.2005**

(86) International application number:
**PCT/JP2005/012998**

(87) International publication number:
**WO 2006/006645 (19.01.2006 Gazette 2006/03)**

(84) Designated Contracting States:
**DE FR GB**

(30) Priority: **14.07.2004 JP 2004207826**

(71) Applicant: **Mycoal Products Corporation**
**Tochigi-chi, Tochigi 328-0067 (JP)**

(72) Inventor: **DODO, Toshihiro,**
**MYCOAL PRODUCTS CORPORATION**
**Tochigi-shi,**
**Tochigi 3280067 (JP)**

(74) Representative: **Thun, Clemens**
**Mitscherlich & Partner**
**Sonnenstrasse 33**
**80331 München (DE)**

(54) **METHOD FOR PRODUCING EXOTHERMAL MIXTURE, EXOTHERMAL MIXTURE, EXOTHERMAL COMPOSITION AND EXOTHERMAL ARTICLE**

(57)     There are provided a process for producing an active heat generating mixture capable of causing an oxidation reaction immediately upon contact with air to generate heat and subsequently initiating an abrupt reaction which after arrival at a certain fixed temperature, is then changed to a mild reaction, the heating generating mixture being cheap and being able to be industrially mass-produced; a heat generating mixture; a heat generating composition; a moldable heat generating composition; and a heat generating body.

The invention is **characterized by** bringing a reaction mixture containing, as essential components, an iron powder, a reaction accelerator and water and having a water content of from 0.5 to 20 % by weight and a water mobility value showing an amount of surplus water of less than 0.01 with an oxidizing gas under circumstances at 0 °C or higher and regulating a temperature rise of the reaction mixture at 1 °C or higher within 10 minutes.

FIG. 2

## Description

[Technical Field]

The present invention relates to a process for producing a heat generating mixture having exothermic characteristics such that it is excellent in exothermic rising properties and having excellent moldability and resistance to compression by bringing at least essential components of a heat generating composition into contact with an oxidizing gas, which is capable of being industrially put into practical use, a heat generating mixture, a heat generating composition and a heat generating body.

[Background Art]

In general, throwaway body warmers are well known as a product to be used by the action of a mixture of an iron powder and a reaction aid, etc. with air (oxygen).

As a metal powder to be used in such a product, it is known that an iron powder is the most general. Salt, water, or the like is used as the reaction aid, and it is also well known that active carbon, vermiculite, diatomaceous earth, wood meal, or a water absorptive polymer is mixed as a water retaining agent for carrying such a substance thereon and used.

In the throwaway body warmer, it is desired that the temperature increases promptly after opening the seal (for example, see Patent Documents 1 and 2).

Patent Document 1 proposes a heat generating composition in which manganese dioxide, cupric oxide and triiron tetroxide are mixed with other heat generating composition components. These substances function as a catalyst and cannot be used as an exothermic substance. Furthermore, in the case where these substances are added in a heat generating composition, the contact between triiron tetroxide, etc. and the surface of an iron powder is not sufficient so that the described effects are not so revealed. In general, in a low temperature region of from about 30 °C to about 90 °C in which a heat generating body is used for the purpose of warming a human body or a body, these substances are not substantially effective in exothermic rising properties, and when the proportion of addition of the substances increases, there was encountered a problem such that the exothermic duration time becomes short.

Patent Document 2 proposes a heat generating body in which after reaching 40 °C, a heat generating composition containing, as major components, iron, water and an oxidation promoter such as salt is accommodated in an air-permeable container. However, it takes 25 minutes for making this heat generating composition reach 40 °C, and therefore, industrial mass production thereof was difficult.

Furthermore, in order to obtain a more comfortable feeling for use, there are proposed a variety of heat generating compositions designing to keep moldability by using a thickener, a binding agent, etc. for the purposes of preventing deviation of a heat generating composition and designing fitness by various shapes. For example, Patent Document 3 proposes a throwaway body warmer comprising a heat generating composition having shape holding properties by adding a powdered thickener such as corn starch and potato starch.

Furthermore, Patent Document 4 proposes a solid heat generating composition prepared by mixing a binding agent such as CMC in a powdered heat generating composition and compression molding the mixture.

Furthermore, Patent Document 5 proposes an ink-like and/or creamy heat generating composition to which viscosity is given by using a thickener and a heat generating body and a process for producing the same.

With respect to such ink-like and/or creamy viscous heat generating compositions or adhesive and flocculating heat generating compositions, in those compositions using a thickener such as glue, gum arabic, and CMC, a flocculant aid such as $\alpha$-starch, an excipient, and a binding agent, particles of the heat generating composition are coupled by such viscosity imparting substances. Thus, though these compositions were excellent with respect to prevention of deviation and moldability, their exothermic performance was remarkably deteriorated. Similarly, in viscous heat generating compositions as prepared by using a thickener or a binding agent, particles of the heat generating composition are coupled by using the thickener or binding agent. Thus, though these compositions were excellent with respect to prevention of deviation and moldability, their exothermic performance was remarkably deteriorated.

That is, even when liberated water is absorbed in a support, a covering material, a water absorptive material, etc., the heat generating composition is viscous due to the binding agent, the thickener, the flocculant aid, the excipient, or the like. Thus, the reaction became slow, or a rapid temperature rise to a prescribed temperature or warming over a long period of time was difficult because the liberated water hardly comes out, or the thickener or the like adversely affects the exothermic substances.

Furthermore, conventionally used powdered or particulate heat generating compositions do not have moldability because surplus water is not present or insufficient. Although these powdered or particulate heat generating compositions are good in exothermic characteristics, since a heat generating body is formed by filling such a composition in an air-permeable accommodating bag, the exothermic temperature distribution is not constant due to deviation of the heat generating composition or the like, the feeling for use is worse, and it is difficult to produce a heat generating body having a shape adaptive to the shape of a body to be heat insulated. Thus, their exothermic performance could not be sufficiently exhibited.

[Patent Document 1] JP-A-53-60885

[Patent Document 2] JP-A-57-10673

[Patent Document 3] JP-A-6-343658

[Patent Document 4] JP-A-59-189183

[Patent Document 5] JP-A-9-75388


[Disclosure of the Invention]


[Problems that the Invention is to Solve]


A problem of the invention is to provide a process for producing an active heat generating mixture capable of causing an oxidation reaction immediately upon contact with air to generate heat and subsequently initiating an abrupt reaction which after arrival at a certain fixed temperature, is then changed to a mild reaction, the heating generating mixture being cheap and being able to be industrially mass-produced; a heat generating mixture; a heat generating composition; a moldable heat generating composition; and a heat generating body.


[Means for solving the Problems]


As set forth in claim 1, a process for producing a heat generating mixture of the invention is characterized by bringing a reaction mixture containing, as essential components, an iron powder, a reaction accelerator and water and having a water content of from 0.5 to 20 % by weight and a water mobility value showing an amount of surplus water of less than 0.01 with an oxidizing gas under circumstances at 0 °C or higher and regulating a temperature rise of the reaction mixture at 1 °C or higher within 10 minutes.

Also, a process for producing a heat generating mixture as set forth in claim 2 is characterized in that in the process for producing a heat generating mixture as set forth in claim 1, an amount of the reaction accelerator is from 2 to 6 parts by weight based on 100 parts by weight of the total sum of the reaction accelerator and water.

Also, a process for producing a heat generating mixture as set forth in claim 3 is characterized in that in the process for producing a heat generating mixture as set forth in claim 1, the reaction mixture is embedded in an air-permeable sheet-like material such as non-woven fabrics and subjected to a contact treatment with the oxidizing gas.

As set forth in claim 4, a heat generating mixture of the invention is characterized by being produced by the production process as set forth in claim 1.

As set forth in claim 5, a heat generating composition of the invention is characterized in that the heat generating mixture as set forth in claim 4 is used as a raw material; the water content is adjusted; an iron powder, a carbon component, a reaction accelerator and water are contained as essential components; and a water mobility value thereof is from 0.01 to 20.

Also, a heat generating composition as set forth in claim 6 is characterized in that in the heat generating composition as set forth in claim 5, the heat generating composition contains at least one member selected from additional components consisting of a water retaining agent, a water absorptive polymer, a pH adjusting agent, a hydrogen formation inhibitor, an aggregate, a fibrous material, a functional substance, a surfactant, an organosilicon compound, a pyroelectric substance, a moisturizer, a fertilizer component, a hydrophobic polymer compound, a heat generating aid, a metal other than iron, a metal oxide other than iron oxide, an acidic substance, and a mixture thereof.

As set forth in claim 7, a heat generating body of the invention is characterized in that the heat generating composition as set forth in claim 5 is accommodated in an air-permeable accommodating bag to form an exothermic part.

Also, a heat generating body as set forth in claim 8 is characterized in that in the heat generating body as set forth in claim 7, the accommodated heat generating composition is a heat generating composition molded body.

Also, a heat generating body as set forth in claim 9 is characterized in that in the heat generating body as set forth in claim 7, the air-permeable accommodating bag is constituted of a substrate and an air-permeable covering material, the heat generating composition is sectioned into plural parts, and the periphery of the heat generating composition is sealed to form plural sectional exothermic parts.

Also, a heat generating body as set forth in claim 10 is characterized in that in the heat generating body as set forth in claim 8, the shape of at least member selected from the heat generating composition molded body, the sectional exothermic part and the exothermic part is a shape selected from the group consisting of a circular shape, an elliptical shape, a polygonal shape, a star shape, and a flower shape with respect to the planar shape and is a shape selected from the group consisting of a polygonal pyramidal shape, a conical shape, a frustum shape, a spherical shape, a parallelepiped shape, a cylindrical shape, a semi-pillar shape, a semicylindroid shape, a semicylidrical shape, a pillar shape, and a cylindroid shape with respect to the three-dimensional shape.

Also, a heat generating body as set forth in claim 11 is characterized in that in the heat generating body as set forth in claim 7, fixing means is provided in at least a part of the exposed surface of the heat generating body.

Also, it is preferable that the heat generating composition does not contain a flocculant aid, a flocculant, an agglomeration aid, a dry binder, a dry binding agent, a dry binding material, a sticky raw material, a thickener, and an excipient.

Also, in the heat generating body, it is preferable that the heat generating composition molded body is subjected to a compression treatment.

Also, in the heat generating body, it is preferable that the fixing means is an adhesive layer and that the adhesive layer contains at least one member selected from additional components consisting of a water retaining agent, a water absorptive polymer, a pH adjusting agent, a surfactant, an organosilicon compound, a hydrophobic polymer compound, a pyroelectric substance, an antioxidant, an aggregate, a carbon component, a fibrous material, a moisturizer, a functional substance, and a mixture thereof.

[Advantages of the Invention]

According to the invention, the following advantages are brought.

1) By using a reaction mixture containing, as essential components, an iron powder, a reaction accelerator and water and having a water content of from 0.5 to 20 % by weight and a water mobility value showing an amount of surplus water of less than 0.01 as a raw material to be used for the contact treatment with an oxidizing gas, reaction activity of the reaction mixture with oxygen is enhanced, whereby a mass production process of an active heat generating composition can be achieved.

2) According to the production process for carrying out the contact treatment with an oxidizing gas according to the invention, a heat generating composition having satisfactory exothermic rising properties can be formed, which is able to be warmed faster 2 times or more as compared with conventional products which are not subjected to a contact treatment with an oxidizing gas.

3) Since the heat generating composition of the invention has satisfactory exothermic rising properties, the amount of use of a carbon component such as active carbon can be reduced by 10 to 20 % or more as compared with conventional products which are not subjected to a contact treatment with an oxidizing gas. Therefore, the invention is advantageous in view of costs.

4) By adjusting the amount of surplus water of the heat generating composition by the water mobility value (for example, at less than 0.01, from 0.01 to 20, or more than 20 but up to 50), various heat generating compositions having satisfactory exothermic rising properties are obtainable. Therefore, various molding methods can be selected, and the production process and utility are widened.

5) The heat generating body using the heat generating composition according to the invention has satisfactory exothermic rising properties. Thus, it is possible to produce a heat generating body having exothermic rising properties such that it is warmed immediately after taking out from an outer bag at the time of use and having an excellent feeling for use such that it is free from an irritated feeling caused due to slow warmth.

6) Furthermore, making the best use of moldability, a heat generating body having two or more plural sectional exothermic parts as disposed and fixed at intervals can be formed; and a heat generating body which is well fitted to a part to be warmed such as the body and is excellent in feeling for use, rich in flexibility and excellent in exothermic rising properties is obtainable.

[Best Modes for Carrying Out the Invention]

Embodiments of a process for producing a heat generating mixture by a contact treatment of a reaction mixture with an oxidizing gas, a heat generating mixture, a heat generating composition, and a heat generating body will be described below in detail.

The invention is to establish a production process capable of achieving mass production by using a reaction mixture containing, as essential components, an iron powder, a reaction accelerator and water and having a water content of from 0.5 to 20 % by weight and a water mobility value showing an amount of surplus water of less than 0.01, thereby raising a reaction rate at the time of a contact treatment with an oxidizing gas and making it possible to achieve a temperature rise of the reaction mixture of 1 °C or higher within a time of 10 minutes. By shortening a time for arrival at a prescribed temperature or higher, it is possible to achieve appropriate activation and prevent unnecessary oxidation on the iron powder from occurring.

Furthermore, a heat generating composition whose water mobility value has been regulated at from 0.01 to 50 by adding a carbon component or the like to a heat generating mixture produced by the contact treatment of the reaction mixture with an oxidizing agent or adjusting the water content is properly sticky, has excellent moldability and is applicable with a molding method such as a force-through molding method and a cast molding method, whereby heat generating bodies having various shapes can be produced. In particular, a heat generating composition having a water mobility value of from 0.01 to 20 is so excellent that it initiates an exothermic reaction immediately upon contact with air, has excellent exothermic rising properties and has excellent moldability.

The contact treatment method of the reaction mixture with an oxidizing gas is not particularly limited so far as it is able to contact treat a reaction mixture containing, as essential components, an iron powder, a reaction accelerator and water and having a water content of from 0.5 to 20 % by weight and a water mobility value of less than 0.01 with an oxidizing gas and regulate a temperature rise of the reaction mixture at 1 °C or more. Specific examples thereof include:

1. a process for producing a heat generating mixture containing an iron powder comprising particles having an iron oxide film on the surface thereof by subjecting a reaction mixture of an iron powder, a reaction accelerator and water in an oxidizing gas atmosphere to a self-exothermic reaction, thereby partially oxidizing the iron powder;
2. a process for producing a heat generating mixture by subjecting a reaction mixture of an iron powder, a reaction accelerator, an acidic substance and water in an oxidizing gas atmosphere to a self-exothermic reaction;
3. a process for producing a heat generating mixture by subjecting a reaction mixture of an iron powder, a reaction accelerator, a carbon component and water in an oxidizing gas atmosphere to a self-exothermic reaction;
4. a process for producing a heat generating mixture by subjecting a reaction mixture of an iron powder, a reaction accelerator, an acidic substance, a carbon component and water in an oxidizing gas atmosphere to a self-exothermic reaction;
5. a process for producing a heat generating mixture containing a partially oxidized iron powder by carrying out the method as set forth above in any one of 1 to 4, wherein the reaction mixture or heat generating mixture as set forth above in any one of 1 to 4 contains a component other than the foregoing components;
6. a process for producing a heat generating mixture by carrying out the method as set forth above in any one of 1 to 5 under circumstances heated so as to have temperature of at least 10 °C higher than the circumferential temperature;
7. a process for producing a heat generating mixture by carrying out the method as set forth above in any one of 1 to 6 by blowing an oxidizing gas;
8. a process for producing a heat generating mixture by carrying out the method as set forth above in 7 by blowing the oxidizing gas heated so as to have a temperature of at least 10 °C higher than the circumferential temperature;
9. a process for producing a heat generating composition by carrying out the method as set forth above in any one of 1 to 8 by contact treating with an oxidizing gas until the temperature exceeds a maximum temperature which is a maximum point of temperature rise by the exothermic reaction;
10. a process for producing a heat generating mixture by carrying out the method as set forth above in any one of 1 to 8 by contact treating with an oxidizing gas until the temperature exceeds a maximum temperature by the exothermic reaction and drops by at least 10 to 20 °C from the maximum temperature;
11. a process for producing a heat generating composition by carrying out the method as set forth above in any one of 1 to 8 by contact treating with an oxidizing gas until the temperature exceeds a maximum temperature which is a maximum point of temperature rise by the exothermic reaction and after intercepting the oxidizing gas, holding it until the temperature of at least the reaction mixture drops by at least 10 to 20 °C from the maximum temperature; and
12. a process for producing a heat generating mixture by heating the reaction mixture or heat generating mixture as set forth above in any one of 1 to 5 under oxidizing gas circumstances while regulating a temperature rise at 1 °C or more.
In addition, a heat generating mixture as prepared by adding other components to the heat generating mixture and further treating with an oxidizing gas may be employed.
Incidentally, the circumstances of the reaction mixture at the time of contact treatment with an oxidizing gas are not limited so far as the reaction mixture is brought into contact with an oxidizing gas under circumstances of 0 °C or higher and a temperature rise of the reaction mixture is regulated at 1 °C within 10 minutes. In the case where the contact treatment is carried out in an open system, the circumstances may be either the state that the reaction mixture is present in a lid-free vessel or the state that an oxidizing gas such as air comes into a vessel through an air-permeable sheet-like material such as non-woven fabrics.
Furthermore, the contact treatment with an oxidizing gas may be carried out with or without stirring in a fluidized or non-fluidized state and may be carried out in a batch or continuous system.
Examples of the final heat generating composition include:

1) a heat generating composition containing, as a heat generating composition raw material, a heat generating mixture produced in the process as set forth above in any one of (1) to (12);
2) a heat generating composition obtained by adding other components to the heating composition as set forth above in 1); and

3) a heat generating composition obtained by adjusting the water content of the heating composition as set forth above in 1) or 2).

The order of the timing of adding other components than the essential components and the timing of adjusting the water content is not limited.

Here, the water content in the reaction mixture or the heat generating mixture prior to the treatment with an oxidizing gas is usually from 0.5 to 20 % by weight, preferably from 1 to 15 % by weight, more preferably from 2 to 10 % by weight, further preferably from 3 to 10 % by weight, and still further preferably from 6 to 10 % by weight.

The temperature of the reaction mixture after the contact with an oxidizing gas is not limited so far as the temperature rise is regulated at 1 °C or more. The temperature of the reaction mixture after the contact with an oxidizing gas is preferably from 1 to 80 °C, more preferably from 1 to 70 °C, further preferably from 1 to 60 °C, and still further preferably from 1 to 40 °C.

The circumferential temperature at the time of contact between the reaction mixture and the oxidizing gas is not limited so far as the temperature of the reaction mixture is raised to a prescribed temperature or higher. The circumferential temperature at the time of contact between the reaction mixture and the oxidizing gas is preferably 0 °C or higher, more preferably from 0 to 250 °C, further preferably from 10 to 200 °C, still further preferably from 20 to 150 °C, even further preferably from 25 to 100 °C, and even still further preferably from 25 to 50 °C.

The time of contact between the reaction mixture and the oxidizing gas is not limited so far as the time required for regulating a temperature rise at 1 °C or more is within 10 minutes. The time of contact between the reaction mixture and the oxidizing gas is preferably from one second to 10 minutes, more preferably from one second to 7 minutes, further preferably from one second to 5 minutes, still further preferably from 2 seconds to 5 minutes, even further preferably from 2 seconds to 3 minutes, and even still further preferably from 2 seconds to one minute.

The temperature of the oxidizing gas is not limited so far as the foregoing circumferential temperature is kept.

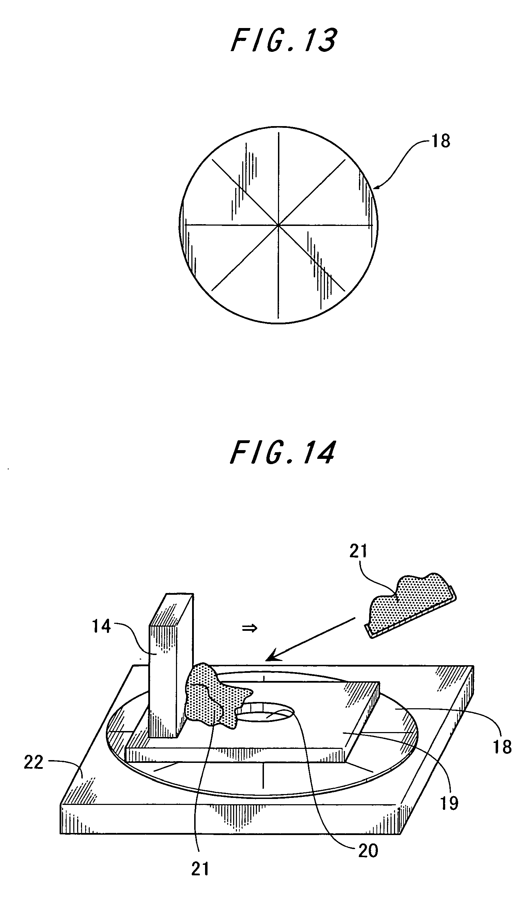

The "water mobility value" as referred to herein is a value showing an amount of surplus water which can transfer to the outside of the heat generating composition in water present in the heat generating composition. This water mobility value will be described below with reference to Figs. 13 to 17.

As shown in Fig. 13, a filter paper 18 of No. 2 (second class of JIS P3801) in which eight lines are drawn radiating from the central point with an interval of 45° is placed on a stainless steel plate 22 as shown in Figs. 14 and 15; a template 19 having a size of 150 mm in length x 100 mm in width and having a hollow cylindrical hole 20 having a size of 20 mm in inner diameter x 8 mm in height is placed in the center of the filter paper 18; a sample 21 is placed in the vicinity of the hollow cylindrical hole 20; and a stuffer plate 14 is moved on and along the template 19 and inserted into the hollow cylindrical hole 20 while stuffing the sample 21, thereby leveling the sample (force-in die molding).

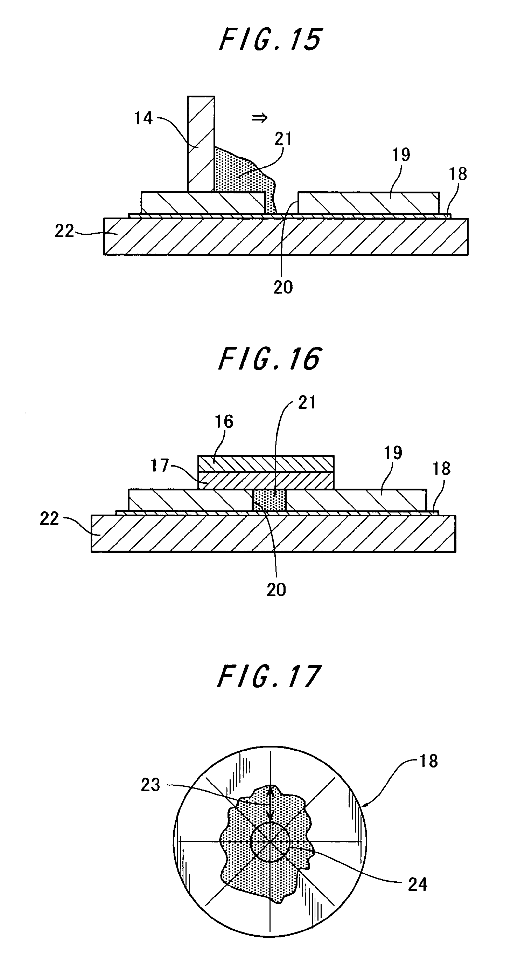

Next, as shown in Fig. 16, a non-water absorptive 70 $\mu$m-thick polyethylene film 17 is placed so as to cover the hole 20, and a flat plate 16 made of stainless steel having a size of 5 mm in thickness x 150 mm in length x 150 mm in width is further placed thereon and held for 5 minutes such that an exothermic reaction is not caused.

Thereafter, a shown in Fig. 17, the filter paper 18 is taken out, and an oozed-out locus of the water or aqueous solution is read as a distance 23 (unit: mm) from a periphery 24 as an edge of the hollow cylindrical hole to an oozed-out tip along the radiating lines. Similarly, a distance 23 from each of the lines is read, and eight values in total are obtained. Each of the eight values (a, b, c, d, e, f, g and h) which are read out is defined as a measured water content value. An arithmetic average value of the eight measured water content values is defined as a water content value (mm) of the sample.

Furthermore, the water content for the purpose of measuring a real water content value is defined as a compounded water content of the heat generating composition corresponding to the weight of the heat generating composition having a size of 20 mm in inner diameter x 8 mm in height or the like, similar measurement is conducted only with water corresponding to that water content, and a value as calculated in the same manner is defined as a real water content value (mm). A value obtained by dividing the water content value by the real water content value and then multiplying with 100 is a water mobility value.

That is, the water mobility value is represented by the following expression.

```
(Water mobility value) = {[Water content value (mm)]/

[(Real water content value (mm))] × 100
```

With respect to the same sample, five points are measured, and the five water mobility values are averaged, thereby defining an average value thereof as a water mobility value of the sample.

Furthermore, in the case of measuring the water mobility value of the heat generating composition in the heat generating body, with respect to the water content for measuring a real water content, a percentage of water content of the heat generating composition is calculated through measurement of the water content of the heat generating composition by an infrared moisture meter, a water content necessary for the measurement is calculated on the basis of the percentage of water content, and a real water content value is measured and calculated from the foregoing water content.

In the invention, the water mobility value (0 to 100) is preferably from 0.01 to 20, more preferably from 0.01 to 18, further preferably from 0.01 to 15, still further preferably from 0.01 to 13, even further preferably from 1 to 13, and even still further preferably from 3 to 13.

A heat generating composition having a water mobility value of less than 0.01 is insufficient in moldability. A heat generating composition having a water mobility value of from 0.01 to 50 has moldability and therefore, is a moldable heat generating composition. When the water mobility value exceeds 20, it is necessary that a part of water of the heat generating composition is removed by water absorption, dehydration, etc. That is, unless a part of water in the heat generating composition molded body is removed by water absorption, dehydration, etc. using a water absorptive packaging material, etc., a practical useful exothermic reaction is not caused. Incidentally, in the case where a water absorptive polymer having a low water absorption speed is used and although a high water mobility value is exhibited at the time of molding, after elapsing a certain period of time, a part of surplus water is taken in the water absorptive polymer, whereby the heat generating composition becomes in an exothermic state with a water mobility value of from 0.01 to 20, even a heat generating composition having a high water mobility value is dealt as a heat generating composition in which surplus water does not function as a barrier layer. In a heat generating composition having a water mobility value exceeding 50, surplus water is too much, the heat generating composition becomes in a slurry state and loses moldability, and the surplus water functions as a barrier layer. Thus, even upon contact with air as it is, an exothermic reaction is not caused.

Furthermore, the "water mobility value" as referred to herein is a value obtained by digitizing surplus water which is the water content capable of being easily and freely oozed out the system in water which is contained in the heat generating composition or mixture or the like. In a mixture in which some components of the heat generating composition or mixture or the like are mixed, the amount of the surplus water is variously changed depending the amount of a component having a water retaining ability such as a water retaining agent, a carbon component and a water absorptive polymer and wettability of each component, and therefore, it is every difficult to predict the water mobility value from the amount of addition of water. Accordingly, since the amount of surplus water of the heat generating composition or mixture of the like is determined from the water mobility value, by determining the amount of addition of water and the amount of other components, a heat generating composition or mixture or the like having a substantially fixed amount of surplus water is obtained with good reproducibility. That is, by previously examining the water mobility value and a composition ratio of a heat generating composition or mixture or the like, a heat generating composition or mixture or the like as compounded along that composition ratio has a water mobility value falling within a fixed range, namely, an amount of surplus water falling within a fixed range. Thus, it is possible to easily produce a variety of heat generating compositions such as a powdered heat generating composition which causes heat generation upon contact with air but does not have moldability, a heat generating composition which causes heat generation upon contact with air and has moldability, and a heat generating composition which, after discharging out a fixed amount of surplus water from the system by water absorption, etc. , causes heat generation upon contact with air and has moldability. Accordingly, if the water mobility value is known, it is possible to note what state does the subject heat generating composition or mixture or the like take.

If the water mobility value is employed, it is possible to embody a desired state with good reproducibility by a simple measurement. Thus, it becomes possible to determine a component ratio of the heat generating composition on the basis of the water mobility value obtained by the measurement and the component ratio, thereby simply achieving actual production of a heat generating composition.

As a use example of the water mobility value, water (or a reaction accelerator aqueous solution) is added to and mixed with a mixture of specified amounts of heat generating composition components exclusive of water (or a reaction accelerator aqueous solution), thereby producing plural heat generating compositions having a different water content. Next, a water mobility value of each of the heat generating compositions is measured, thereby determining a relationship between the amount of addition of water (or a reaction accelerator aqueous solution) and a water mobility value.

A heat generating composition which has moldability and causes heat generation upon contact with air has a water mobility value of from 0.01 to 20. By determining a compounding ratio of the respective components therefrom to prepare a mixture in this compound ratio, a moldable heat generating composition in which water does not function as a barrier layer and which has moldability causes heat generation upon contact with air can be produced with good reproducibility. In this way, since surplus water is used as a connecting substance and a flocculant aid or a dry binding material is not used, reaction efficiency of the iron powder does not drop. Thus, an exothermic performance can be obtained in a small amount as compared with the case of using a flocculant aid or a dry binding material.

Incidentally, in the invention, what water does not function as a barrier layer and causes an exothermic reaction upon contact with air means that water in a heat generating composition does not function as a barrier layer which is an air intercepting layer and immediately after the production of a heat generating composition, comes into contact with air, thereby immediately causing an exothermic reaction.

By using a moldable heat generating composition containing this surplus water as a connecting substance, it becomes possible to produce, for example, a super thin and super flexible heat generating body having plural sectional exothermic parts of a heat generating composition molded body on a substantially planar substrate in a maximum width of preferably from 1 to 50 mm, and more preferably from 1 to 20 mm, or in a maximum diameter of preferably from 1 to 50 mm, and more preferably from 1 to 20 mm (in the case where two or more axes are present as in an ellipse, the major axis is dealt as a length, while the minor axis is dealt as a width).

The "surplus water" as referred to herein means water or an aqueous solution portion which is present excessively in the heat generating composition and easily transfers to the outside of the heat generating composition. The surplus water is defined as a water mobility value which is a value of water or a value of an aqueous solution portion sucked out from the heat generating composition, etc. by a filter paper. When the heat generating composition has an appropriate amount of surplus water, it is assumed that the surplus water causes hydration against hydrophilic groups in the components of the heat generating composition due to a bipolar mutual action or hydrogen bond, etc. and that it is present even in the surroundings of hydrophobic groups while having high structural properties.

This is connecting water as a connecting substance in some meaning. Besides, there is water in a state called as free water which can freely move. When the surplus water increases, the structure is softened, and the free water is found.

The "moldability" as referred to in the invention exhibits that a molded body of the heat generating composition having a cavity or concave die shape is formed by force-through molding using a trimming die having a cavity or cast molding using a concave die, whereby after molding including mold release, the molding shape of the heat generating composition molded body is held.

When the moldability is revealed, since the shape is held until the heat generating composition molded article is at least covered by a covering material and a seal part is formed between the substrate and the covering material, sealing can be achieved in the periphery of the shape with a desired shape. Also, since so-called "spots" which are a collapsed piece of the heat generating composition are not scattered in the seal part, the sealing can be achieved without causing cutting in seal. The presence of the spots causes insufficient sealing.

1) Measurement device:

With respect to the measurement device, a stainless steel-made molding die (a plate having a size of 2 mm in thickness x 200 mm in length x 200 mm in width and having a cavity as treated by R5 in four corners of 60 mm in length x 40 mm in width in a central part thereof) and a fixable leveling plate are disposed above a travelable endless belt, and magnets (two magnets having a size of 12.5 mm in thickness x 24 mm in length x 24 mm in width are disposed in parallel) are disposed under the endless belt.

The magnets should cover a region of the leveling plate and the vicinity thereof and a region larger than a region covered by a cut side (40 mm) vertical to the advancing direction of the cavity of the molding die.

2) Measurement method:

With respect to the measurement method, a stainless steel plate having a size of 1 mm in thickness x 200 mm in length x 200 mm in width is placed on the endless belt of the measurement device, a polyethylene film having a size of 70 μm in thickness x 200 mm in length x 200 mm in width is placed thereon, and a stainless steel-made molding die is further placed thereon.

Thereafter, a leveling plate is fixed in a position of the cavity of the molding die of 50 mm far from the end portion in the advancing direction of the endless belt, 50 g of a heat generating composition is then placed in the vicinity of the leveling plate between the leveling plate and the cavity, and the heat generating composition is filled in the cavity of the molding die while leveling it by moving the endless belt at 1.8 m/min. After the molding die has completely passed through the leveling plate, the traveling of the endless belt is stopped. Next, the molding die is removed, and a heat generating composition molded body as laminated on the polyethylene film is observed.

3) Judgment method:

With respect to the judgment method, in the surroundings of the heat generating composition molded body, in the case where any collapsed piece of the heat generating composition molded body exceeding a maximum length of 800 μm is not present and the number of collapsed pieces of the heat generating composition molded body having a maximum length of from 300 to 800 μm is not more than 5, it is to be noted that the heat generating composition has moldability.

The moldability is an essential property for a heat generating composition to be used in the molding system. If the heat generating composition does not have moldability, it is impossible to produce a heat generating body by the molding system.

The "moldable heat generating composition" as referred to herein means a heat generating composition which contains, as essential components, an iron powder, a reaction accelerator and water, does not contain a flocculant aid, a flocculant, an agglomeration aid, a dry binder, a dry binding agent, a dry binding material, a sticky binder, a thickener, and an excipient, has surplus water so as to have a water mobility value of from 0.01 to 20 and has moldability by the surplus water as a connecting substance; in which the water in the heat generating composition does not function as a barrier layer; and which causes an exothermic reaction upon contact with air.

The "resistance to compression" as referred to herein means that a heat generating composition compressed body obtained by compressing a heat generating composition molded body having a heat generating composition accommodated in a molding die within the die to such an extent that the heat generating composition compressed body having a thickness of 70 % of the die thickness holds 80 % or more of exothermic rising properties of the exothermic rising properties of the heat generating composition molded body prior to the compression (a difference in temperature between one minute and 3 minutes after starting a heat generation test of the heat generating composition).

Here, the measurement method of exothermic rinsing properties for the resistance to compression will be described below.

1. Heat generating composition molded body:

1) A magnet is provided in the vicinity of a central part of the back side of a polyvinyl chloride-made supporting plate (3 mm in thickness x 600 mm in length x 600 mm in width) of a footed supporting table so as to cover a cavity shape of a molding die.

2) A temperature sensor is placed on the central part the surface of the supporting plate.

3) A polyethylene film (25 μm in thickness x 250 mm in length x 200 mm in width) as provided with an adhesive layer having a thickness of about 80 μm is stuck onto the supporting plate via a sticky layer such that the center of the polyethylene film is positioned at the sensor.

4) On an underlay plate (280 mm in length x 150 mm in width x 50 μm to 2 mm in thickness), a polyethylene film (230 mm in length x 155 mm in width x 25 μm to 100 μm in thickness) is placed such that one end of the polyethylene film is projected by about 20 mm outside the underlay plate and that one end thereof in the length direction is substantially coincident with one end of the underlay plate.

5) A template (230 mm in length x 120 mm in width x 3 mm in thickness) having a cavity (80 mm in length x 50 mm in width x 3 mm in height) is placed on the polyethylen film placed on the underlay plate; a template is placed on the polyethylene film such that one end thereof in the length direction is fitted to one end where the underlay plate and the polyethylene film are coincident with each other and that in the width direction, one end part of the width of the template is placed at a position of the central part by about 20 mm far from an opposing end to the side where the polyethylene film is projected outward from the underlay plate. Next, the resulting assembly is placed on the supporting plate together with the underlay plate.

6) A sample is placed in the vicinity of the cavity; a force-in die plate is moved along the molding die; the sample is charged into the cavity while stuffing; and the sample is leveled while stuffing along the template plane (force-in die molding), thereby filling the sample in the die.

7) Next, the magnet beneath the supporting plate is removed; the end portion of the projected polyethylene film is pressed; the underlay plate is removed; and the temperature measurement is started.

2. Heat generating composition compressed body:

1) to 6) are the same as in the case of the heat generating composition molded body.

8) A die having a convex having a thickness of 0.9 mm which can substantially tightly come into the cavity in relation of the cavity with an unevenness is fitted to the cavity and compressed by a roll press or plate press to prepare a heat generating composition compressed body having a thickness of 2.1 mm (compressed to 70 % of the die thickness) within the die.

9) The resulting assembly is placed on the supporting plate together with the underlay plate; the magnet beneath the supporting plate is removed; the end portion of the projected polyethylene film is pressed; the underlay plate is removed; and the temperature measurement is started.

With respect to the measurement of the exothermic temperature, the temperature is measured for 5 minutes at a measurement timing of 2 seconds using a data collector, and resistance to compression is judged in terms of a difference in temperature between after elapsing one minute and after elapsing 3 minutes.

The thickness after compression is preferably from 50 to 99.5 %, more preferably from 60 to 99.5 %, and further preferably from 60 to 95 % of the die thickness.

With respect to the particle size of the water-insoluble solid component, separation is conducted using a sieve, and the particle size of the component which has passed through the sieve is calculated from an opening of the sieve. That is, sieves of 8, 12, 20, 32, 42, 60, 80, 100, 115, 150, 200, 250 and 280 meshes and a receiving dish are combined in this order from up to down. About 50 g of water-insoluble solid component particles are placed on the uppermost 8-mesh sieve and shaken for one minute using an automatic shaker. Weights of the water-insoluble solid component particles on each of the sieves and the receiving dish are weighed. The total amount thereof is defined as 100 %, and the particle size distribution is determined from weight fractions. When the sum of all receiving dishes under the sieve of a specific mesh size becomes 100 % which is the total sum of the particle size distribution, the size ($\mu$m) calculated from the opening of the specific mesh is defined as the particle size of the water-insoluble solid component. Incidentally, each of the mesh sieves may be combined with other mesh sieves.

Here, the particles which have passed through a 16-mesh sieve are defined to have a particle size of not more than 1 mm; the particles which have passed through a 20-mesh sieve are defined to have a particle size of not more than 850 $\mu$m; the particles which have passed through a 48-mesh sieve are defined to have a particle size of not more than 300 $\mu$m; the particles which have passed through a 60-mesh sieve are defined to have a particle size of not more than 250 $\mu$m; the particles which have passed through a 65-mesh sieve are defined to have a particle size of not more than 200 $\mu$m; the particles which have passed through an 80-mesh sieve are defined to have a particle size of not more than 180 $\mu$m; the particles which have passed through a 100-mesh sieve are defined to have a particle size of not more than 150 $\mu$m; the particles which have passed through a 115-mesh sieve are defined to have a particle size of not more than 120 $\mu$m; the particles which have passed through a 150-mesh sieve are defined to have a particle size of not more than 100 $\mu$m; and the particles which have passed through a 250-mesh sieve are defined to have a particle size of not more 63 $\mu$m, respectively. The same is applicable to mesh sizes of less than these mesh sizes.

As the "oxidizing gas" as referred to herein, any gas can be used as the oxidizing gas so far as it is oxidizing. Examples thereof include an oxygen gas, air, and mixed gases of an inert gas (for example, a nitrogen gas, an argon gas, and a helium gas) and an oxygen gas.

Although the mixed gas is not limited so far as it contains oxygen, mixed gases containing 10 % or more of an oxygen gas are preferable, and of these, air is especially preferable.

If desired, a catalyst such as platinum, palladium, iridium, and mixtures thereof can also be used.

The oxidation reaction can be carried out under stirring in an oxidizing gas atmosphere optionally under a pressure and/or upon irradiation of ultrasonic waves.

The optimal condition of the oxidation reaction may be properly experimentally determined.

An amount of the oxidizing gas to be used is not limited but may be adjusted depending upon the kind of the oxidizing gas, the kind and particle size of the iron powder, the water content, the treatment temperature, the treatment method, and the like.

In the case of an open system, there is no limitation so far as a necessary amount of oxygen can be taken in. In order to prevent fly of the reaction mixture or contamination of dusts, etc., the system may be surrounded by an air-permeable raw material such as non-woven fabrics and woven fabrics. So far as the system is in an air-permeable state, it is to be noted that the system is an open system.

In the case where air is used in the system of blowing an oxidizing gas, for example, the amount of air is preferably from 0.01 to 1, 000 L/min, more preferably from 0.01 to 100 L/min, and further preferably from 0.1 to 50 L/min per 200 g of the iron powder under one atmosphere. In the case of other oxidizing gas, the amount of the oxidizing gas may be reduced into the concentration of oxygen on the basis of the case of air.

If desired, a peroxide may be added. Examples of the peroxide include hydrogen peroxide and ozone.

Here, so far as the iron powder is partially oxidized, the state of the reaction mixture or heat generating mixture at the time of the contact treatment with an oxidizing gas may be any of a standing state, a transfer state, or a fluidizing state by stirring, etc. and may be properly selected. Furthermore, the circumstances at the time of mixing the respective components of the reaction mixture, the heat generating mixture or the heat generating composition and at the time of the contact treatment with a mixed oxidizing gas at the time of adjusting the water content are not limited, and examples thereof include those in an oxidizing gas atmosphere and those in blowing of an oxidizing gas.

As a method for measuring a temperature rise of the heat generating composition, a heat generating composition is allowed to stand in a state that it is sealed in an air-impermeable outer bag for one hour under a condition that the circumferential temperature is 20 $\pm$ 1 °C.

1) A magnet is provided in the vicinity of a central part of the back side of a polyvinyl chloride-made supporting plate (5 mm in thickness x 600 mm in length x 600 mm in width) of a footed supporting table so as to cover a cavity shape of a molding die.

2) A temperature sensor is placed above the central part of the supporting plate.

3) A polyethylene film having a size of 25 μm in thickness x 250 mm in length x 200 mm in width as provided with an adhesive layer having a thickness of about 80 μm is stuck onto the supporting plate via a sticky layer such that the center of the polyethylene film is positioned at the sensor.

4) The heat generating composition is taken out from the outer bag.

5) A template having a size of 250 mm in length x 200 mm in width) having a cavity (80 mm in length x 50 mm in width x 3 mm in height) is placed above the central part of the polyethylene film; a sample is placed in the vicinity of the cavity; a force-in die plate is moved along the molding die; the sample is charged into the cavity while stuffing; and the sample is leveled while stuffing along the template plane (force-in die molding), thereby filling the sample in the die. Next, the magnet beneath the supporting plate is removed, and the temperature measurement is started. With respect to the measurement of the exothermic temperature, the temperature is measured for 10 minutes at a measurement timing of 2 seconds using a data collector, and exothermic rising properties are judged in terms of the temperature after elapsing 3 minutes.

The heat generation test of the heat generating body follows the JIS temperature characteristic test.

The iron powder is not limited, and examples thereof include cast iron powders, atomized iron powders, electrolyzed iron powders, reduced iron powders, sponge iron powders, and iron alloy powders thereof. In addition, the iron powder may contain carbon and oxygen, and an iron powder containing 50 % or more of iron and other metals may be employed. The kind of the metal which is contained as an alloy, etc. is not particularly limited so far as the iron component works as a component of the heat generating composition. Examples of such a metal include metals such as aluminum, manganese, copper, nickel, silicon, cobalt, palladium, and molybdenum, and semiconductors. The metal of the invention includes a semiconductor. Such a metal or alloy may be contained only in the surface or the interior, or may be contained in both the surface and the interior.

An iron powder containing a carbon component and/or covered by a carbon component is also preferable as the iron powder. Although a proportion of the carbon component is not limited so far as a ratio of the iron component to the carbon component is 50 % by weight or more, an iron powder in which the surface thereof is partially covered by from 0.3 to 3.0 % by weight of a conductive carbonaceous substance is useful. Examples of the conductive carbonaceous substance include carbon black, active carbon, carbon nanotubes, carbon nanohorns, and fullerens. Ones which have become conductive by doping are also employable. Examples of the iron powder include reduced iron powders, atomized iron powders, and sponge iron powders. In particular, the case where the conductive carbonaceous substance is active carbon and the iron powder is a reduced iron powder is useful for a heat generating body.

In the iron powder of the invention, the content of the metal other than iron is usually from 0.01 to 50 % by weight, and preferably from 0.1 to 10 % by weight based on the whole of the iron powder. Furthermore, in order to efficiently carry out covering by a conductive carbonaceous substance, an oil such as a spindle oil may be added in an amount of from 0.01 to 0.05 % by weight to such an extent that the fluidity of the iron powder is not hindered.

The iron powder in the heat generating composition may also be calculated from the iron powder in the reaction mixture or heat generating mixture.

The "iron oxide film" as referred to herein is a film made of oxygen-containing iron such as iron oxide, hydroxide or oxyhydroxide. Furthermore, in the invention, it is thought that the active iron powder forms an iron oxide film at least locally on the surface of the iron powder, from which an oxidation reaction promoting effect is obtained by a local cell as formed between an iron matrix and an iron oxide film or a pit inside and outside the iron oxide film.

Although the mechanism is not elucidated in detail, it is assumed that upon contact between the oxidizing gas and the components, not only an iron oxide film, namely, an oxygen-containing film is formed on the surface of the iron powder due to the oxidation of the components, especially the oxidation of the iron powder, but also the surface of active carbon is oxidized and/or the oxidized iron component is adhered, whereby hydrophilicity is imparted or improved, and coupling between the components or structurization takes place through the mediation of water.

That is, it is assumed that some kind of a change in the function occurs such that an iron oxide film is formed on the surface of the iron powder, the shape of the iron powder particle becomes irregular, a strain is generated due to the oxidation, or a water-containing pit is formed, whereby the iron powder is activated and exothermic rising properties are improved.

Furthermore, the case where magnetite ($Fe_3O_4$) is present in the iron oxide film is preferable because the conductivity is excellent, and the case where hematite ($Fe_2O_3$) is present in the iron oxide film is also preferable because the iron oxide film becomes porous. Moreover, it is assumed that the carbon component is oxidized on the surface thereof and becomes a carbon component which is rich in oxides on the surface thereof, whereby the hydrophilicity increases and the activity increases.

The reaction accelerator is not particularly limited so far as it is able to promote the reaction of the heat generating substance. Examples thereof include metal halides, nitrates, acetates, carbonates, and metal sulfates. Examples of metal halides include sodium chloride, potassium chloride, magnetic chloride, calcium chloride, ferrous chloride, ferric chloride, sodium bromide, potassium bromide, ferrous bromide, ferric bromide, sodium iodide, and potassium iodide. Examples of nitrates include sodium nitrate and potassium nitrate. Examples of acetates include sodium acetate. Examples of carbonates include ferrous carbonate. Examples of metal sulfates include potassium sulfate, sodium sulfate, and ferrous sulfate.

As the water, one from a proper source may be employed. Its purity and kind and the like are not particularly limited.

In the case of the heat generating composition, the content of water is preferably from 1 to 60 % by weight, more preferably from 1 to 40 % by weight, further preferably from 7 to 40 % by weight, still further preferably from 10 to 35 % by weight, and even further preferably from 20 to 30 % by weight of the heat generating composition.

Furthermore, in the case of the reaction mixture or heat generating mixture prior to the contact treatment with an oxidizing gas, the content of water is preferably from 0.5 to 20 % by weight, more preferably from 1 to 20 % by weight, further preferably from 5 to 20 % by weight, and still further preferably from 7 to 15 % by weight of the reaction mixture or heat generating mixture.

In the case of measuring the water mobility value of the heat generating composition in the heat generating body and the thickness and amount of wustite of the iron oxide film of iron powder in the mixture or the heat generating composition in the heat generating body, the heat generating composition or mixture may be measured according to the following items.

1) Water mobility value:

The heat generating composition is taken out from the heat generating body and measured according to the foregoing method of measuring a water mobility value.

2) Thickness and amount of wustite of iron oxide film of iron powder:

A measuring sample as prepared by dispersing the heat generating composition, the heat generating composition molded body, the heat generating composition compression molded body or the mixture in nitrogen-purged ion-exchanged water in a nitrogen atmosphere, separating the iron powder using a magnet and drying the iron powder in a nitrogen atmosphere is used.

It is to be noted that the heat generating composition molded body includes a heat generating composition compressed body, except for the case where the heat generating composition molded body and the heat generating composition compressed body are distinguishingly used.

Furthermore, in the reaction mixture and the heat generating mixture of the invention, although there is no particular limitation for the compounding ratio thereof, it is preferred that the amount of the reaction accelerator is from 1.0 to 5.0 parts by weight and the amount of water is from 0.5 to 20 parts by weight based on 100 parts by weight of the iron powder (in the case of an iron powder having an iron oxide film, an iron powder as reduced in terms of an iron component amount). In addition, it is preferred that the amount of the carbon component is from 1.0 to 50 parts by weight; the amount of the water retaining agent is from 0.01 to 10 parts by weight; the amount of the water absorptive polymer is from 0.01 to 20 parts by weight; the amount of the pH adjusting agent is from 0.01 to 5 parts by weight; the amount of the hydrogen formation inhibitor is from 0.01 to 12 parts by weight; the amount of each of the aggregate, the fibrous material and the functional substance is from 0.01 to 10 parts by weight; the amount of the surfactant is from 0.01 to 5 parts by weight; the amount of each of the organosilicon compound, the pyroelectric substance, the moisturizer, the fertilizer component, the hydrophobic polymer compound, the anti-foaming agent, the heat generating aid, the metal other than iron and the metal oxide other than iron oxide is from 0.01 to 10 parts by weight; and the amount of the acidic substance is from 0.001 to 1 parts by weight, respectively.

As the anti-foaming agent, there are enumerated not only usual pH adjusting agents such as poly(sodium phosphate) but also those which are used in this field.

The reaction mixture of the invention contains, as essential components, an iron powder, a reaction accelerator and water and may further contain at least one member selected from additional components consisting of a carbon component, a water retaining agent, a water absorptive polymer, a pH adjusting agent, a hydrogen formation inhibitor, an aggregate, a fibrous material, a functional substance, a surfactant, an organosilicon compound, a pyroelectric substance, a moisturizer, a fertilizer component, a hydrophobic polymer compound, a heat generating aid, a metal other than iron, a metal oxide other than iron oxide, an acidic substance, and a mixture thereof.

The carbon component is not particularly limited so far as it contains carbon as a component. Examples thereof include carbon black, graphite, active carbon, carbon nanotubes, carbon nanohorns, and flullerenes. Carbon which has become conductive by doping or the like is also employable. There are enumerated active carbons as prepared from coconut shell, wood, charcoal, coal, bone carbon, etc. and carbons as prepared from other raw materials such as animal products, natural gases, fats, oils, and resins. In particular, active carbons having an adsorption retaining ability are preferable.

Furthermore, it is not always required that the carbon component is present alone. In the case where an iron powder containing the carbon component and/or covered by the carbon component is used in the heat generating composition, it is to be noted that the heat generating composition contains the carbon component even though the carbon component is not present alone.

The water retaining agent is not limited so far as it is able to retain water. Examples thereof include porous materials derived from plants having high capillary function and hydrophilicity such as wood meal, pulp powder, active carbon, saw dust, cotton cloth having a number of cotton fluffs, short fiber of cotton, paper dust, and vegetable materials, water-containing magnesium silicate based clay minerals such as active clay and zeolite, pearlite, vermiculite, silica based porous substances, coralline stone, and volcanic ash based substances (for example, terraballoon, *shirasu* balloon, and taisetsu balloon). In order to increase a water retaining ability and enhance a shape holding ability of such a water retaining agent, the water retaining agent may be subjected to a processing treatment such as baking and/or pulverization.

The water absorptive polymer is not particularly limited so far as it is a resin having a crosslinking structure and having a water absorption magnification of ion-exchanged water of 3 times or more of the dead weight. Furthermore, a water absorptive polymer the surface of which is crosslinked may be employed. Conventionally known water absorptive polymers and commercial products may also be employed.

Examples of the water absorptive polymer include poly(meth)acrylic acid crosslinked materials, poly(meth)-acrylic acid salt crosslinked materials, sulfonic group-containing poly(meth)acrylic ester crosslinked materials, polyoxyalkylene group-containing poly(meth)acrylic ester crosslinked materials, poly(meth)acrylamide crosslinked materials, crosslinked materials of a copolymer of a (meth)acrylic acid salt and a (meth)acrylamide, crosslinked materials of a copolymer of a hydroxyalkyl (meth) acrylate and a (meth)acrylic acid salt, polydioxolane crosslinked materials, crosslinked polyethylene oxide, crosslinked polyvinylpyrrolidone, sulfonated polystyrene crosslinked materials, crosslinked polyvinylpyridine, saponification products of a starch-poly (meth) acrylonitrile graft copolymer, starch-poly (meth)acrylic acid (salt) graft crosslinked copolymers, reaction products of polyvinyl alcohol and maleic anhydride (salt), crosslinked polyvinyl alcohol sulfonic acid salts, polyvinyl alcohol-acrylic acid graft copolymers, and polyisobutylene maleic acid (salt) crosslinked polymers. These water absorptive polymers may be used alone or in combination with two or more kinds thereof.

Of these water absorptive polymers, water absorptive polymers having biodegradation properties are not limited so far as they are a biodegradable water absorptive polymer. Examples thereof include polyethylene oxide crosslinked materials, polyvinyl alcohol crosslinked materials, carboxymethyl cellulose crosslinked materials, alginic acid crosslinked materials, starch crosslinked materials, polyamino acid crosslinked materials, and polylactic acid crosslinked materials.

The pH adjusting agent is not limited so far it is able to adjust the pH. Examples thereof include alkali metal weak acid salts and hydroxides and alkaline earth metal weak acid salts and hydroxides such as $Na_2CO_3$, $NaHCO_3$, $Na_3PO_4$, $Na_2HPO_4$, $Na_5P_3O_{10}$, $NaOH$, $KOH$, $Ca(OH)_2$, $Mg(OH)_2$, and $Ca_3(PO_4)_2$.

The hydrogen formation inhibitor is not limited so far as it is able to inhibit the formation of hydrogen. Examples thereof include one member or two or more members selected from the group consisting of sulfur compounds, oxidizing agents, alkaline substances, sulfur, antimony, selenium, phosphorus, and tellurium. Incidentally, examples of sulfur compounds include compounds with an alkali metal or an alkaline earth metal, metal sulfides such as calcium sulfide, metal sulfites such as sodium sulfite, and metal thiosulfates such as sodium thiosulfate.

The alkaline substance is not limited so far as it is a substance exhibiting alkalinity. Examples thereof include silicates, phosphates, sulfites, thiosulfates, carbonates, hydrogencarbonates, hydroxides, $Na_3PO_4$, and $Ca(OH)_2$.

The aggregate is not limited so far as it is useful as a filler and/or is useful for making the heat generating composition porous. Examples thereof include fossilized coral (for example, coral fossil and weathered coral fossil), bamboo charcoal, bincho charcoal, silica-alumina powders, silica-magnesia powders, kaolin, crystalline cellulose, colloidal silica, pumice, silica gel, silica powders, mica powders, clays, talc, synthetic resin powders or pellets, foamed synthetic resins such as foamed polyesters or polyurethanes, diatomaceous earth, alumina, and cellulose powder. Incidentally, it is to be noted that kaolin and crystalline cellulose are not contained in the heat generating composition of the invention but contained only in the case of using as an adhesive.

The fibrous material is an inorganic fibrous material and/or an organic fibrous material. Examples thereof include rock wool, glass fibers, carbon fibers, metal fibers, pulps, papers, non-woven fabrics, woven fabrics, natural fibers such as cotton and hemp, regenerated fibers such as rayon, semi-synthetic fibers such as acetates, synthetic fibers, and pulverized products thereof.

The functional substance is not limited so far as it is a substance having any function. Examples thereof include at least one member selected from minus ion emitting substances and far infrared ray radiating substances.

The minus ion emitting substance is not limited so far as it emits a minus ion as a result either directly or indirectly, and examples thereof include ferroelectric substances such as tourmaline, fossilized coral, granite, and calcium strontium propionate, and ores containing a radioactive substance such as radium and radon.

The far infrared ray radiating substance is not limited so far as it radiates far infrared rays. Examples thereof include ceramics, alumina, zeolite, zirconium, and silica.

The surfactant includes anionic surfactants, cationic surfactants, nonionic surfactants, and ampholytic surfactants. Especially, nonionic surfactants are preferable, and examples thereof include polyoxyethylene alkyl ethers, alkylphenol·ethylene oxide adducts, and higher alcohol phosphoric acid esters.

The organosilicon compound is not limited so far as it is a compound having at least an Si-O-R bond and/or an Si-N-R bond and/or an Si-R bond. The organosilicon compound is in the form of a monomer, a lowly condensed product, a polymer, etc. Examples thereof include organosilane compounds such as methyltriethoxysilane; and dimethylsilicone oil, polyorganosiloxane, or silicone resin compositions containing the same.

The pyroelectric substance is not limited so far as it has pyroelectricity. Examples thereof include tourmaline, hemimorphic ores, and pyroelectric ores. Tourmaline or achroite which is a kind of tourmaline is especially preferable. Examples of the tourmaline include dravite, schorl, and elbaite.

The moisturizer is not limited so far as it is able to hold moisture. Examples thereof include hyaluronic acid, collagen, glycerin, and urea.

The fertilizer component is not limited so far as it is a component containing at least one of three elements of nitrogen, phosphorus and potassium. Examples thereof include a bone powder, urea, ammonium sulfate, calcium perphosphate, potassium chloride, and calcium sulfate.

The hydrophobic polymer compound is not limited so far as it is a polymer compound having a contact angle with water of 40° or more, preferably 50° or more, and more preferably 60° or more in order to improve the draining in the composition. The shape of the hydrophobic polymer compound is not limited, and examples thereof include powdery, particulate, granular, and tablet shapes. Examples of the hydrophobic polymer compound include polyolefins such as polyethylene and polypropylene, polyesters, and polyamides.

Examples of the heat generating aid include metal powders, metal salts, and metal oxides such as Cu, Mn, $CuCl_2$, $FeCl_2$, manganese dioxide, cupric oxide, triiron tetroxide, and mixtures thereof.

The acidic substance may be any of an inorganic acid, an organic acid, or an acidic salt. Examples thereof include hydrochloric acid, sulfuric acid, nitric acid, acetic acid, oxalic acid, citric acid, malic acid, maleic acid, chloroacetic acid, iron chloride, iron sulfate, iron oxalate, iron citrate, aluminum chloride, ammonium chloride, and hypochlorous acid.

The heat generating composition of the invention contains, as essential components, an iron powder, a carbon component, a reaction accelerator and water and may further contain at least one member selected from additional components consisting of a water retaining agent, a water absorptive polymer, a pH adjusting agent, a hydrogen formation inhibitor, an aggregate, a fibrous material, a functional substance, a surfactant, an organosilicon compound, a pyroelectric substance, a moisturizer, a fertilizer component, a hydrophobic polymer compound, a heat generating aid, a metal other than iron, a metal oxide other than iron oxide, an acidic substance, and a mixture thereof.

The heat generating composition of the invention contains, as essential components, an iron powder, a carbon component, a reaction accelerator and water, and its production process is one which can be put into practical use on an industrial scale. A reaction mixture containing, as essential components, an iron powder, a reaction accelerator and water and having a water content of from 1 to 20 % by weight and a water mobility value showing a surplus water content of less than 0.01 is brought into contact with an oxidizing gas under circumstances at 0 °C or higher, a temperature rise of the reaction mixture is regulated at 1 °C or more within 10 minutes to produce a heat generating mixture, and the subject heat generating mixture is used as a raw material to form a heat generating composition. Alternatively, a heat generating composition may be formed by subsequently further adjusting the water content, or by further adding a carbon component, etc. and adjusting the water content.

In the invention, it has become possible to realize the contact treatment with an oxidizing gas within a short period of time by regulating the water content of the reaction mixture at a fixed amount or less, especially regulating the surplus water content of the reaction mixture at a fixed amount or less and carrying out an oxidizing contact treatment. By specifying the surplus water content and performing the treatment within a short period of time, adverse influences such as poor initial exothermic rising of the heat generating composition and shortening of the heat generation-retaining time can be avoided. Thus, it has become possible to establish an industrial mass-production process. Furthermore, although stirring or the like may not be achieved during the contact treatment with an oxidizing gas, when stirring or the like is achieved, the contact treatment with an oxidizing gas can be surely carried out.

Here, so far as the iron powder is partially oxidized, the state of the reaction mixture or heat generating mixture at the time of the contact treatment with an oxidizing gas may be any of a standing state, a transfer state, or a fluidizing state by stirring, etc. and may be properly selected. Furthermore, the circumstances at the time of mixing the respective components of the reaction mixture, the heat generating mixture or the heat generating composition and at the time of mixing at the time of adjusting the water content are not limited, and examples thereof include those in an oxidizing gas atmosphere and those in blowing of an oxidizing gas.

The "adjustment of the water content" as referred to herein means that after contact treating the heat generating mixture with an oxidizing gas, water or an aqueous solution of a reaction accelerator is added. Although the amount of addition of water or an aqueous solution of a reaction accelerator is not limited, examples thereof include the addition of a weight corresponding to a reduced weight by the contact treatment and the addition of a weight such that a desired water mobility value is obtained.

Whether or nor the adjustment of the water content is introduced may be properly determined depending upon the utility.

The heat generating composition of the invention contains, as essential components, an iron powder, a carbon component, a reaction accelerator and water and is started from a mixture obtained by contact treating a reaction mixture containing, as essential components, an iron powder, a reaction accelerator and water with an oxidizing gas. The heat generating composition of the invention is usually one obtained by adjusting the water content of a heat generating mixture and is a heat generating composition which is satisfactory in the exothermic rising, has a suitable amount of surplus water and has excellent moldability. Furthermore, it is possible to produce a heat generating body which can become promptly warm at the time of use.

Accordingly, at least the iron powder further including the carbon component has a history of oxidation by the contact treatment with an oxidizing gas, and it is thought that this is deeply related to excellent exothermic rising properties, exothermic endurance and excellent moldability.

Furthermore, the resistance to compression is preferably 80 % or more, more preferably 85 % or more, and further preferably 90 % or more.

When the iron powder which is contact treated with an oxidizing gas according to the invention is used, the amount of addition of the carbon component (for example, active carbon) in the heat generating composition can be reduced by, for example, 20 % or more. By reducing the amount of addition of the carbon component, the costs are lowered.

Furthermore, in the heat generating composition of the invention, although there is no particular limitation for the compounding ratio thereof, it is preferred that the amount of the carbon component is from 1.0 to 50 parts by weight; the amount of the reaction accelerator is from 1.0 to 5.0 parts by weight; and the amount of water is from 5 to 60 parts by weight, respectively based on 100 parts by weight of the iron powder (in the case of an iron powder having an iron oxide film, an iron powder as reduced in terms of an iron component amount in the reaction mixture). In addition, it is preferred that the amount of the water retaining agent is from 0.01 to 10 parts by weight; the amount of the water absorptive polymer is from 0.01 to 20 parts by weight; the amount of the pH adjusting agent is from 0.01 to 5 parts by weight; the amount of the hydrogen formation inhibitor is from 0.01 to 12 parts by weight; the amount of each of the aggregate, the fibrous material and the functional substance is from 0.01 to 10 parts by weight; the amount of the surfactant is from 0.01 to 5 parts by weight; the amount of each of the organosilicon compound, the pyroelectric substance, the moisturizer, the fertilizer component, the hydrophobic polymer compound, the anti-foaming agent, the heat generating aid, the metal other than iron and the metal oxide other than iron oxide is from 0.01 to 10 parts by weight; and the amount of the acidic substance is from 0.001 to 1 parts by weight, respectively.

According to the process for producing a heat generating mixture of the invention, it is possible to obtain a heat generating composition having excellent exothermic rising properties, excellent hydrophilicity and excellent moldability. In particular, by employing the water mobility value of from 0.01 to 50 jointly, a heat generating composition having both remarkably excellent moldability and exothermic characteristics is obtained.

Since the heat generating composition as produced by the production process of the invention is remarkably improved with respect to exothermic rising properties, the amount of addition of a carbon component such as active carbon in the heat generating composition can be reduced by, for example, 20 % or more, leading to contribution to a reduction in costs. Furthermore, since the hydrophilicity is remarkably improved, the moldability with a mold is remarkably improved.

Thus, since after molding, collapsed pieces of the heat generating composition are not scattered on the surroundings of the heat generating composition molded body, sealing can be appropriately achieved so that a heat generating body which is free from cutting in seal can be produced. In this way, heat generating composition molded bodies of various shapes can be produced, and heat generating bodies of various shapes can be formed.

Furthermore, since a marketed heat generating body in which a heat generating composition is accommodated in an accommodating bag is provided on the assumption that it is accommodated in an outer bag which is an air-impermeable accommodating bag and is storable over a long period of time, it is preferred to use a heat generating composition containing a hydrogen formation inhibitor. Since the heat generating composition which has passed through the contact treatment with an oxidizing gas is an active composition, it is important that the heat generating composition contains a hydrogen formation inhibitor. Also, this efficacy is further strengthened by using a pH adjusting agent together.

Furthermore, so far as the reaction characteristics and exothermic characteristics are not affected, the heat generating composition having a water mobility value of less than 0.01 may contain a flocculant aid, a flocculant, an agglomeration aid, a dry binder, a dry binding agent, a dry binding material, a sticky raw material, a thickener, an excipient, or a water-soluble polymer in an amount ranging from 0.01 to 3 parts by weight respectively.

The "flocculant aid" as referred to herein is a flocculant aid as described in Japanese Patent No. 3,161,605 (JP-T-11-508314) such as gelatin, natural gum, and corn syrup.

The "flocculant" as referred to herein is a flocculant as described in JP-T-2002-514104 such as corn syrup and maltitol syrup.

The "agglomeration aid" as referred to herein is an agglomeration aid as described in JP-T-2001-507593 such as corn syrup.

The "dry binder" as referred to herein is a dry binder as described in JP-T-2002-514104 such as microcrystalline cellulose, maltodextrin, and mixtures thereof.

The "dry binding agent" as referred to herein is a dry binding agent as described in JP-T-2001-507593 such as maltodextrin and sprayed lactose.

The "dry binding material" as referred to herein is a dry binding material as described in JP-T-11-508314 such as microcrystalline cellulose, maltodextrin, and mixtures thereof.

The "sticky raw material" or the "binder" as referred to herein is a sticky raw material or binder as described in JP-A-4-293989 such as water glass, polyvinyl alcohol (PVA), and carboxymethyl cellulose (CMC).

The "thickener" as referred to herein is a thickener as described in JP-A-6-343658 such as corn starch and potato starch.

The "excipient" as referred to herein is an excipient as described in JP-A-7-194641 such as $\alpha$-starch and sodium alginate.

As the "water-soluble polymer" as referred to herein, the water-soluble polymer in the adhesive layer can be used.

Furthermore, in the case where an exothermic part is constituted of the heat generating composition molded body, the particle size of a solid component constituting each of the reaction mixture, the heat generating mixture and the heat generating composition is not limited so far as the heat generating composition has moldability. In the case where any one of the sizes (length, width and height) of the heat generating composition molded body is made small, it is preferred to make the particle size of the solid component small.

A maximum particle size of the water-insoluble solid component exclusive of the reaction accelerator and water in the components constituting the heat generating composition or the like is preferably not more than 2.5 mm, more preferably not more than 930 $\mu$m, further preferably not more than 500 $\mu$m, still further preferably not more than 300 $\mu$m, even further preferably not more than 250 $\mu$m, and even still further preferably not more than 200 $\mu$m. Moreover, 80 % or more of the particle size of the solid component is preferably not more than 500 $\mu$m, more preferably not more than 300 $\mu$m, further preferably not more than 250 $\mu$m, still further preferably not more than 150 $\mu$m, and even further preferably not more than 100 $\mu$m.

Furthermore, the heat generating composition can be classified into a powder or granulate heat generating composition (having a water mobility value of less than 0.01), a moldable heat generating composition (having a water mobility value of from 0.01 to 20), and a sherbet-like heat generating composition (having a water mobility value exceeding 20 but not more than 50) depending upon the state of adjustment of the water content or the amount of surplus water. The heat generating composition as classified depending upon the water mobility value is as above.

The water-soluble polymer compound is not limited so far as it is a water-soluble organic polymer. Examples thereof include single kinds or combinations of two or more kinds such as starch, gum arabic, methyl cellulose (MC), carboxymethyl cellulose (CMC), sodium carboxymethyl cellulose, polyvinyl alcohol, gelatin, polyacrylic acid, polyacrylic acid salts, polyacrylic acid partially neutralized products, polyvinylpyrrolidone, and N-vinylacetamide copolymers.

Next, the heat generating body will be described.

The heat generating body of the invention is a heat generating body in which a heat generating composition having satisfactory exothermic rising properties is accommodated in an accommodating bag in which at least a part thereof is air permeable.

In addition, a preferred heat generating body of the invention may form an exothermic part by one section or may form an exothermic part from a sectional exothermic part composed of two or more plural sections as disposed at intervals.

Furthermore, for the storage or transportation, the heat generating body of the invention is sealed in an outer bag which is an air-impermeable accommodating bag.

The air-permeable accommodating bag which is used in the invention is not particularly limited with respect to the material quality and construction of a packaging material so far as it is able to hold the heat generating composition therein, is free from leakage of the raw materials during the use of the heat generating body, has a strength such that there is no possibility of breakage of the bag, and has air permeability necessary for the heat generation.

Furthermore, air permeability of the accommodating bag can be provided in a part or on one surface or both surfaces of the bag, and the air-permeable surface can be constituted of an air-permeable packing material. In the case where the both surfaces are air permeable, the air permeability of one surface may differ from that of the other surface.

Usually, the accommodating bag of the invention comprises a substrate and a covering material, and an underlay material may further be provided between the substrate and the covering material.

The air-permeable accommodating bag of the invention is not particularly limited with respect to the material quality and construction of a packaging material so far as it is able to hold the mixture therein, is free from leakage of the raw materials during the use of the heat generating body, has a strength such that there is no possibility of breakage of the bag, and has air permeability necessary for the heat generation.

Furthermore, with respect to the air permeability of the accommodating bag, an air-permeable packaging material can be used in a part or on one surface or both surfaces of the bag.

The air permeability is not limited so far as the heat generation can be kept. In the case of use as a usual heat generating body, the air permeability is usually from 50 to 10, 000 $g/m^2/24$ hr, preferably from 50 to 5, 000 $g/m^2/24$ hr, more preferably from 70 to 5, 000 $g/m^2/24$ hr, further preferably from 700 to 1,000 $g/m^2/24$ hr, and still further preferably from 80 to 800 $g/m^2/24$ hr in terms of a moisture permeability by the Lyssy method. Here, the term "$g/m^2/24$ hr" has the same meaning as "$g/(m^2/24$ hr)".

When the moisture permeability is less than 50, the heat value is small and a sufficient thermal effect is not obtained, and therefore, such is not preferable. On the other hand, when it exceeds 10,000 $g/m^2/24$ hr, the exothermic temperature is high so that a problem in safety may possibly be generated, and therefore, such is not preferable.

However, there is no limitation even when the moisture permeability exceeds 10,000 $g/m^2/24$ hr depending upon the utility, or even in use at a moisture permeability closed to the open system, according to circumstances.

The air-permeable raw material constituting the air-permeable accommodating bag is not particularly limited so far as it can be formed into a film and is able to realize air permeability by stretching and/or extraction with a soluble filling agent or a method such as perforation by an extra fine needle.

Examples thereof include air-permeable films (for example, porous films and perforated films); materials having air permeability by themselves (for example, papers and non-woven fabrics) ; materials prepared by laminating at least one of papers and air-permeable films and non-woven fabrics so as to have air permeability; materials prepared by providing an air-impermeable packaging material comprising a non-woven fabric having a polyethylene film laminated thereon with fine pores by using a needle, etc. so as to have air permeability; non-woven fabric whose air permeability is controlled by laminating a fiber and heat bonding under pressure; porous films; and materials prepared by sticking a non-woven fabric onto a porous film.

The porous film can be properly selected among porous films obtained by stretching a film made of a polyolefin based resin (for example, polyethylene, linear low density polyethylene, and polypropylene) or a fluorine based resin (for example, polytetrafluoroethylene) and a filler.

The "perforated film" as referred to herein is a film prepared by providing an air-impermeable film (for example, polyethylene films) with fine pores by using a needle so as to have air permeability.

The packaging material of the accommodating bag may be of a single-layered structure or a multilayered structure.

Its structure is not limited, and the multilayered structure is enumerated as follows.

The substrate is made of layer A/layer B, layer A/layer B/layer C, or layer A/layer B/layer C/layer D; and the covering material is made of layer F/layer G, layer E/layer F/layer G, or layer F/layer H/layer G.

Examples of the layer A include thermoplastic resin films (for example, polyethylene), heat seal layers (for example, polyethylene, EVA, and a mixture of EVA and polyethylene), and water absorptive papers.

Examples of the layer B include non-woven fabrics of a thermoplastic resin (for example, nylons), non-water absorptive papers, water absorptive papers, thermoplastic resin films (for example, polyethylene films, polypropylene films, polyester films, and polyamide (for example, nylons) films), wicks (for example, non-water absorptive papers and water absorptive papers).

Examples of the layer C include adhesive layers, non-water absorptive papers, water absorptive papers, thermoplastic resin films (for example, polyethylene), non-slip layers, and non-woven fabrics of a thermoplastic resin (for example, polyesters and nylons).

Examples of the layer D include separators, thermoplastic resin films (for example, polyethylene), and non-woven fabrics.

Examples of the layer E include heat seal layers.

Examples of the layer F include porous films or perforated films made of a thermoplastic resin (for example, polyethylene), films made of a thermoplastic resin (for example, polyethylene), non-water absorptive papers, and water absorptive papers.

Examples of the layer G include non-woven fabrics of a thermoplastic resin (for example, polyesters and nylons).

Examples of the layer H include non-water absorptive papers and water absorptive papers.

Examples of the substrate or covering material include polyethylene-made heat seal layer/polypropylene film, EVA-made heat seal layer/polypropylene film, EVA-made heat seal layer/polypropylene film/adhesive layer/separator, EVA-made heat seal layer/polyethylene film/nylon non-woven fabric, polyethylene-made heat seal layer/polypropylene film/polypropylene non-woven fabric, non-woven fabric/porous film, non-woven fabric/paper or perforated (provided by a needle or laser) film/porous film, non-woven fabric/paper or porous film/perforated (provided by a needle or laser) film, and non-woven fabric/paper or porous film/non-woven fabric. A method for laminating the respective layers is not limited.

The respective layers may be directly laminated; the respective layers may be laminated via an air-permeable adhesive layer or a laminating agent layer; and the respective layers may be laminated by hot melt extrusion or the like. In the invention, it is to be noted that polyethylene produced by using a metallocene catalyst is also included in the polyethylene.

The heat generating body may be accommodated in an outer bag which is an air-impermeable accommodating bag, stored and transported. The outer bag is not limited so far as it is air-impermeable and may be made of a laminate. Examples thereof include air-impermeable raw materials such as nylon, polyester and polypropylene films which are subjected to a moisture-proof treatment with OPP, CPP, polyvinylidene chloride, metal oxides (including semiconductors) such as aluminum oxide and silicon oxide, etc., aluminum foils, and aluminum-deposited plastic films.

Examples of the air-impermeable raw material include polyethylene, polypropylene, cellophane, polyesters, polyamides, polyvinyl alcohol, polyvinyl chloride, polyvinylidene chloride, polyurethane, polystyrene, ethylene-vinyl acetate copolymers, polycarbonates, and hydrochlorinated rubber. Of these, polyolefin based resin-made raw materials are preferable because a uniform air-permeable film is obtainable by stretching, etc. Examples of this polyolefin based resin include homopolymers (for example, polyethylene, polypropylene, and polybutadiene) or copolymers or blended polymers thereof.

Furthermore, among the foregoing air-impermeable raw materials, examples of a film having high air impermeability include films in which a single layer or multiple layers made of a metal including semiconductors or a compound thereof are provided on an air-impermeable raw material film. Examples of the metal including semiconductors include silicon, aluminum, titanium, tin, indium, and alloys or mixtures of these metals.

Examples of the metal compound including semiconductors include oxides, nitrides and oxynitrides of the foregoing metals or alloys or mixtures.

Examples of the layer include a silicon oxide layer, an aluminum oxide layer, a silicon oxynitride layer, and layers obtained by laminating arbitrary layers thereof.

Furthermore, there are also numerated layers obtained by laminating a stretched polyolefin film (for example, a biaxially stretched polypropylene film) on the foregoing layer.

Examples of the heat generating body which is accommodated in an outer bag include a heat generating body in which a produced heat generating body is sealed between two air-impermeable films or sheets.

The raw material constituting the substrate, the covering material or the underlay material is not limited so far as it functions as an accommodating bag of the heat generating composition, and any materials which are hitherto used in air-permeable accommodating bags of heat generating bodies are useful.

Examples thereof include air-impermeable raw materials, air-permeable raw materials, water absorptive raw materials (for example, papers and rayon), non-water absorptive raw materials, stretchable raw materials, non-stretchable raw materials, foamed raw materials, and heat sealable raw materials. The raw material may be properly used in a desired shape such as a film, a sheet, a non-woven fabric, a woven fabric, and a composite thereof depending upon the intended utility.

The non-water absorptive raw material is not limited so far as it is non-water absorptive. Examples thereof include films, sheets and coatings made of a synthetic resin (for example, polyethylene, polypropylene, nylons, polyacrylates, polyesters, polyvinyl alcohol, and polyurethane) or the foregoing hydrophobic polymer.

The stretchable packaging material is not particularly limited so far as it is stretchable. That is, it is only required that the stretchable packaging material is stretchable as a whole. The stretchable packaging material may be formed of a single material or a composite material among stretchable substrates or a combination of a stretchable substrate and a non-stretchable substrate.

Examples of the stretchable packaging material include fabrics, films, spandex yarns, yarns, strands, flat plates, ribbons, slit films, foamed bodies or non-woven fabrics constituted of a single material (for example, natural rubbers, synthetic rubbers, elastomers, and stretchable shape memory polymers) or a mixed material, a blended material or a combination thereof with a non-stretchable raw material, and composite stretchable materials made of a laminate among such stretchable raw materials or such a stretchable raw material and a non-stretchable material.

As the fiber constituting the woven fabric, natural fibers, regenerated fibers using a natural raw material such as viscose fibers, semi-synthetic fibers, synthetic fibers, and mixtures of two or more kinds thereof can be used. These fibers can also be used as a fibrous material.

The non-woven fabric is not limited so far as it is a cloth-like sheet as produced by welding a fiber by tanglement or bonding by thermal, chemical, physical or mechanical means or other means, such as heat, an adhesive, and high-pressure stream of water. Materials having one kind or a combination of two or more kinds of stretchable, non-stretchable, water absorptive, non-water absorptive, heat sealable, and non-heat sealable properties are employable. Examples thereof include vegetable fibers (for example, rayon, nylons (polyamides), polyesters, polyacrylates, polypropylene, vinylon, polyethylene, polyurethane, cupra, cotton, cellulose, and pulp), single fibers or composite fibers or mixed fibers made of a synthetic pulp and a thermoplastic polymer substance, and mixtures thereof. Single non-woven fabrics or laminates of mixed or accumulated fiber layers of such a fiber are used. Short fiber non-woven fabrics, long fiber non-woven fabrics, and continuous filament non-woven fabrics can also be used. Furthermore, from the standpoint of production process, dry non-woven fabrics, wet non-woven fabrics, spunbonds, spunlaces, and the like can be used. Incidentally, the stretchable non-woven fabric is not limited so far as it is stretchable. Examples thereof include non-woven fabrics of an elastomer based rubber based fiber, entangled fabrics of a polyolefin based or polyester based crimped fiber, and stretchable non-woven fabrics or polyurethane based non-woven fabrics produced by using a binder or by a hot melt measure.

Non-woven fabrics made of a composite fiber of a core-sheath structure are also employable.

A basis weight of the non-woven fabric is not limited but is preferably from 10 to 200 g/m$^2$.

The paper is not limited so far as it is water absorptive and is usually used. Examples thereof include papers and cardboards. Examples include one kind or laminates of two or more kinds of thin papers (for example, blotting paper, tissue paper, and crepe paper), packaging papers (for example, craft paper), miscellaneous papers (for example, card paper), corrugated cardboard, inner wicks for corrugated cardboard (for example, pulp wick and special wick), liners for corrugated cardboard (for example, kraft and jute), cardboards (for example, coated cardboard), and construction papers (for example, gypsum board original paper).

The non-water absorptive paper is not limited so far as it is non-water absorptive.

Examples thereof include papers obtained by subjecting the foregoing paper or cardboard to a non-water absorptive treatment. Examples include papers resulting from a non-water absorptive treatment by impregnation or coating with an oil or a synthetic resin and papers resulting from laminating a non-water absorptive raw material such as a polyethylene film.

If desired, the paper and the non-water absorptive paper may be subjected to waterproof processing or may be provided with perforations using laser, a needle, or the like so as to adjust or provide air permeability.

Examples of the foamed raw material include foamed bodies such as sheets formed of at least one kind selected from foamed polyurethane, foamed polystyrene, foamed ABS resins, foamed polyvinyl chloride, foamed polyethylene, and foamed polypropylene.

The heat sealable raw material may be a single raw material or a composite raw material having a heat seal layer and is not limited so far as at least a part thereof can be welded by heating.

Examples of the heat sealable raw materials or a hot melt based adhesive constituting a heat seal layer include polyolefins (for example, polyethylene and polypropylene) or olefin copolymer resins, ethylene based hot melt resins such as ethylene-acrylic acid ester copolymer resins (for example, ethylene-methyl methacrylate copolymers, ethylene-methyl methacrylic acid-acrylic acid ester copolymers, ethylene-α-olefin copolymers, ethylene-vinyl acetate copolymer resins, and ethylene-isobutyl acrylate copolymer resins), polyamide based hot melt resins, polyester based hot melt resins, butyral based hot melt resins, cellulose derivative based hot melt resins, polymethyl methacrylate based hot melt resins, polyvinyl ether based hot melt resins, polyurethane based hot melt resins, polycarbonate based hot melt resins, hot melt based resins (for example, polyvinyl acetate and vinyl chloride-vinyl acetate copolymers), and films or sheets thereof. Furthermore, the hot melt based resins or films or sheets thereof can be compounded with various additives such as antioxidants. In particular, low density polyethylene, polyethylene obtained by using a metallocene catalyst, and ethylene-α-olefin copolymers are useful. The α-olefin is not limited so far as it is a monomer having a double bond in the terminal thereof. Examples thereof include polypropylene, 1-butene, 1-heptane, 1-hexene, 1-octene, and 4-methyl-1-ptentene.

Furthermore, in the components and packaging materials such as the substrate, the covering material, and the underlay material, which are used in the heat generating composition and the heat generating body of the invention, besides conventionally used raw materials, biodegradable raw materials can be used.

In the substrate, the covering material, the air-permeable adhesive layer, the underlay material, and the heat generating composition containing the heat generating composition molded body, at least the heat generating composition may be subjected to a compression treatment.

In particular, a molded body prepared by appropriately compressing the heat generating composition molded body of the invention under pressure is markedly improved in shape holding properties. For example, even when a perforated film which is difficult with respect to the pressure adjustment is used as a raw material of the air-permeable part in place of the porous film, or even when an inner pressure of the accommodating bag becomes equal to or more than the outer pressure, shape collapse hardly occurs so that a perforated film can be used. Accordingly, not only the range for selecting an air-permeable raw material is widened so that the costs can be lowered, but also a body to be warmed can be uniformly warmed at an appropriate temperature over a long period of time.

A heat generating composition molded body prepared by compression treating the heat generating composition of the invention is a non-elastic body. The resistance to compression of the heat generating composition is preferably 80 % or more, more preferably 85 % or more, and further preferably 90 % or more, or it may exceed 100 %.

In the exothermic part, for the purpose of containing a magnetic substance in at least a part thereof or one sectional exothermic part to improve the blood circulation or stiff shoulders due to a magnetic effect, it is also possible to accommodate therein a magnetic substance such as a magnet. In the case of a heat generating body of a plural-section type, it is preferable that the magnetic substance is accommodated in at least one sectional region of the sectional regions.

In order to achieve the separation easily, the separator may be provided with a notch such as a slit. The adhesive layer is preferably non-transferable.

At least one member selected from additional components consisting of a moisturizer, a functional substance, an aggregate, a pyroelectric substance, a magnetic body, and a mixture thereof may be contained in or carried on at least one member selected from the substrate, the underlay material, the covering material, and the adhesive layer. The content of such a material is not particularly limited. However, from the viewpoints of medicinal effect, economy, adhesive force, and the like, it is preferably from 0.01 to 25 parts by weight, and more preferably from 0.5 to 15 parts by weight based on 100 parts by weight of the adhesive.

A hot melt based adhesive may be provided between the hydrophilic adhesive layer and the substrate or the covering material. Furthermore, in the case of providing the hydrophilic adhesive in the heat generating body, though there is no limitation, it is preferred to provide the hydrophilic adhesive in the heat generating body after the seal treatment of the heat generating body.

With respect to the exothermic part of the invention, the exothermic part may be formed by one section. Alternatively, sectional exothermic parts may be formed by disposing and fixing two or more plural sections at intervals, and an exothermic part may be formed from a gathering of these sectional exothermic parts and provided for the exothermic part.

Furthermore, in the case of an exothermic part having a sectional exothermic part, the size of the heat generating composition molded body on the substrate is not larger than that of the sectional exothermic part, and the periphery of the heat generating composition molded body is heat sealed, thereby constituting the sectional exothermic part and the exothermic part. A capacity of the sectional exothermic part or the exothermic part is composed of a capacity of the filling heat generating composition or a capacity of the heat generating composition molded body and a spacial capacity surrounding it. A capacity ratio of the capacity of the filling heat generating composition or the heat generating composition molded body to the capacity of the sectional exothermic part or the capacity of the exothermic part is usually from about 0. 3 to about 1.0, preferably from about 0.35 to about 1.0, more preferably from about 0.5 to about 1.0, further preferably from about 0.7 to about 1.0, still further preferably from about 0.8 to about 1.0, and even further preferably from about 0.9 to about 1.0.

From the viewpoint of flexibility of the heat generating body, in the case of forming the sectional exothermic part, when its size is small as far as possible, it is possible to make the heat generating body flexible as a whole. In a plan view, it is preferable that one side of a sectional exothermic part which is constituted of at least two sides having a different length from each other is short as far as possible. Also, with respect to a sectional exothermic part which is constituted of the same side as in a square or the like or one diameter as in a circle or the like, it is preferable that the longest length is short as far as possible.

The sectional exothermic part accommodates therein a heat generating composition or a heat generating composition molded body as sectioned by a sectioning part which is a seal part.

In the sectional exothermic part or the heat generating composition molded body of the invention, its maximum width is usually from 0.5 to 60 mm, preferably from 0.5 to 50 mm, more preferably from 1 to 50 mm, further preferably from 3 to 50 mm, still further preferably 3 to 30 mm, even further preferably from 5 to 20 mm, even still further preferably from 5 to 15 mm, and most preferably from 5 to 10 mm. Furthermore, its maximum height is usually from 0.1 to 30 mm, preferably from 0.1 to 10 mm, more preferably from 0.3 to 10 mm, further preferably from 1 to 10 mm, and still further preferably from 2 to 10 mm. Moreover, its longest length is usually from 5 to 300 mm, preferably from 5 to 200 mm, more preferably from 5 to 100 mm, further preferably from 20 to 150 mm, and still further preferably from 30 to 100 mm.

A capacity of the sectional exothermic part or a volume of the heat generating composition molded body is usually from 0.015 to 500 cm$^3$, preferably from 0.04 to 30 cm$^3$, more preferably from 0.1 to 30 cm$^3$, further preferably from 1 to 30 cm$^3$, and still further preferably from 3 to 20 cm$^3$.

In the sectional exothermic part, when the sectional exothermic part which is an accommodating region of the heat generating composition is filled with the heat generating composition molded body, a volume ratio of the volume of the heat generating composition molded body which is an occupying region of the heat generating composition molded body to the capacity of the sectional exothermic part which is an accommodating region of the heat generating composition is usually from 0. 6 to 1, preferably from 0.7 to 1, more preferably from 0.8 to 1, and further preferably from 0.9 to 1.0.

Furthermore, a width of the sectioned part which is a space between the sectional exothermic parts is not limited so far as sectioning can be achieved. It is usually from 0.1 to 50 mm, preferably from 0.3 to 50 mm, more preferably from 0.3 to 50 mm, further preferably from 0. 3 to 40 mm, still further preferably from 0.5 to 30 mm, even further preferably from 1.0 to 20 mm, and even still further preferably from 3 to 10 mm.

Incidentally, the heat generating composition molded body or the sectional exothermic part may have any shape. The shape may be a planar shape, and examples thereof include a circular shape, an elliptical shape, a polygonal shape, a star shape, and a flower shape. Also, the shape may be a three-dimensional shape, and examples thereof include a polygonal pyramidal shape, a conical shape, a frustum shape, a spherical shape, a parallelepiped shape, a cylindrical shape, a semi-pillar shape, a semicylindroid shape, a semicylidrical shape, a pillar shape, and a cylindroid shape. Furthermore, in these shapes, the corner may be rounded, thereby processing the corner in a curvilinear or curved state, or the central part may be provided with a concave.

Furthermore, the "volume of the heat generating composition molded body of the invention" as referred to herein means a volume of the heat generating composition molded body or compressed heat generating composition molded body. Furthermore, the "capacity of the sectional exothermic part" as referred to herein means an internal capacity of the sectional exothermic part having a heat generating composition molded body accommodated therein.

Furthermore, in the shape of each of the heat generating composition molded body, the sectional exothermic part and the exothermic part, the height of the central part may become low step by step toward the surroundings, namely a height gradation may be provided, or a reverse height gradation may be provided.

In the case of a molding system, with respect to the molding order, the size of the heat generating composition molded body is first determined, and the size of the sectional exothermic part is then determined.

In the sectional exothermic part, the accommodating bag, the outer bag (accommodating bag of the heat generating body), and the like, packaging materials or the like constituting the same are sealed in the sectioned part or its surroundings. Its seal is not limited but is properly selected depending upon the desire. As one example, sealing is carried out in a point-like (missing line) state or entirely by compression seal (adhesive seal), warm compression seal (adhesive seal), bonding seal, heat bonding seal, heat melt seal (heat seal), etc. by means of pressurizing, warming, heating or a combination thereof via an adhesive layer and/or a bonding agent layer and/or a heat seal layer. Selection of any one or a combination of these methods may be made depending upon the desire.
In this way, it is possible to seal and form a sectional exothermic part, an inner bag (accommodating bag), an outer bag, etc. Sewing processing can also be employed as one of seal means.

In the above, a width of the periphery in the substrate for forming an accommodating bag such as a substrate and a covering material or in the seal part of the sectioned part can be properly determined. It is usually not more than 50 mm, preferably from 1 to 30 mm, and more preferably from 3 to 20 mm.

In the sectioned part, a cutting line such as a perforation can be provided as the need arises. This perforation may be provided to a degree such that bending properties are improved or may be provided to a degree such that cutting by hand is possible, for example, a degree for forming a heat generating body of a size adaptive with a place for application of a human body, or the like. This degree is not limited but is determined depending upon the desire.

A heat generating body in which a number of sectional exothermic parts are continuously provided at intervals and the perforation is provided to a degree such that cutting by hand is possible in the sectioned exothermic part can be cut into an appropriate size at the time of use on the basis of the purpose for use adaptive with a place for application of a human body, or the like and applied.
In that case, the size of the heat generating body and the size and number of the sectional exothermic parts may be properly set up. There are no limitations regarding such size and number. Furthermore, the sectioned part can be formed in arbitrary directions such as a length or width direction, length and width directions, and an oblique direction.

Furthermore, at least a part of the surface of the heat generating composition may be covered by an air-permeable adhesive layer of a network-like polymer, etc. Also, an underlay material such as non-woven fabrics may be provided between the air-permeable adhesive layer and the covering material.

Furthermore, the entire surface or a partial surface of at least one kind of the heat generating composition molded body, the substrate, the covering material and the underlay material may be subjected to a pressurizing treatment or the like or may be formed with irregularities. In this way, transfer of the laminate between the substrate and the covering material may be prevented.

The "perforation" as referred to in the invention includes one which is intermittently cut for the purpose of improving flexural properties of the sectioned part and one which is intermittently cut such that cutting by hand is possible. Its degree, length and aperture are not limited but are determined depending upon the desire. The perforation may be provided in all sectioned parts or may be partially provided.
The shape is not particularly limited, and examples thereof include a circle, an ellipse, a rectangle, a square, and a cut line (linear shape). For example, in the perforation which is intermittently cut such that cutting by hand is possible, a circular hole having an aperture of from $\phi$10 to 1,200 $\mu$m can be enumerated. The aperture of the hole is more preferably from $\phi$20 to 500 $\mu$m.
It is preferable that the holes are positioned lined up in the length and width. Furthermore, a shortest space between outer peripheries of the adjacent holes in the length and width is not limited so far as it is satisfactory with flexural properties and possibility of cutting by hand. The shortest space is preferably from 10 to 2,000 $\mu$m, more preferably from 10 to 1,500 $\mu$m, further preferably from 20 to 1,000 $\mu$m, still further preferably from 20 to 500 $\mu$m, and even further preferably from 20 to 200 $\mu$m. The cutting properties by hand are remarkably improved by a balance between the aperture of the hole and the shortest space of outer peripheries of the adjacent holes in the length and width.
The hole may be a cut line, and its length may be a length corresponding to the aperture or may be larger than the aperture. A shortest space between ends of the adjacent cut lines in the length and width is corresponding to the shortest space between outer peripheries of the adjacent holes.
For example, an aperture of the hole of from $\phi$10 to 2, 000 $\mu$m is corresponding to a length of from 10 to 2,000 $\mu$m, and a shortest space between outer peripheries of the adjacent holes in the length and width of from 10 to 2,000 $\mu$m is corresponding to a shortest space between ends of the adjacent cut lines in the length and width of from 10 to 2,000 $\mu$m.

In the case of a cut line, since it becomes long in one direction, its length can be prolonged and may be from 10 to 50, 000 μm. A shortest distance between the adjacent cut lines in the length and width may be from 1 to 5,000 μm.

The fixing means is not limited so far as it has capability for fixing a thermal packaging body for joint surroundings or a material having an exothermic part to a prescribed part.

As the fixing means, an adhesive layer, a hook and eye, a hook and button, a hook and loop fastener such as Velcro, a magnet, a band, a string, and combination thereof can be arbitrarily used.

Incidentally, in the case of a band, fixing means for adjustment may be further constructed by a combination of a hook and loop fastener and an adhesive layer.

Here, the "hook and loop fastener" as referred to herein has a fastening function by a combination of a loop as a female fastener with a male fastener capable of fastening the female fastener thereto, which is known as trade names such as Magic Tape (a registered trademark), Magic Fastener (a registered trademark), Velcro Fastener, and Hook and Loop Tape. Examples of the material having a loop function include non-woven fabrics and woven fabrics of napped or hole-containing yarns. Such a material having a loop function (female fastener function) may be covered on the surface of a paddling forming the band, or the band may be constructed of such a material itself. Although the hook member which is the male fastener member is not particularly limited, examples thereof include hook members formed of a polyolefin based resin (for example, polyethylene and polypropylene), a polyamide, a polyester, etc. Although the shape of the hook is not particularly limited, a hook having a cross-sectional shape such as an I type, an inverted L type, an inverted J type, and a so-called mushroom type is preferable because it is easily hooked by the loop and does not give an extreme stimulus to the skin. Incidentally, the hook may be adhered to the entire area of a fastening tape, and only the hook may be used as a fastening tape while omitting a tape substrate.

The adhesive layer may contain at least one member selected from additional components consisting of a water retaining agent, a water absorptive polymer, a pH adjusting agent, a surfactant, an organosilicon compound, a hydrophobic polymer compound, a pyroelectric substance, an antioxidant, an aggregate, a fibrous material, a moisturizer, a functional substance, and a mixture thereof.

The adhesive of the invention is classified into a non-hydrophilic adhesive, a mixed adhesive, and a hydrophilic adhesive (for example, a gel).

The adhesive constituting the adhesive layer is not limited so far as it has an adhesive strength necessary for adhering to the skin or clothes. Adhesives of every form such as a solvent based adhesive, an aqueous adhesive, an emulsion type adhesive, a hot melt type adhesive, a reactive adhesive, a pressure-sensitive adhesive, a non-hydrophilic adhesive, and a hydrophilic adhesive are employable.

The adhesive layer includes one layer of a non-hydrophilic adhesive constituted of the non-hydrophilic adhesive and non-hydrophilic adhesive layers constituted of the non-hydrophilic adhesive.

It is to be noted that a material whose water absorption properties are improving by containing a water absorptive polymer or a water retaining agent in the non-hydrophilic adhesive layer is dealt as the non-hydrophilic adhesive layer.

A hot melt based adhesive may be provided between the hydrophilic adhesive layer and a substrate or a covering material. Furthermore, in the case where the hydrophilic adhesive is provided in a thermal packaging body for joint surroundings, there is no limitation. After seal treating a thermal packaging body for joint surroundings, a hydrophilic adhesive layer may be provided in the thermal packaging body for joint surroundings.

Furthermore, the adhesive layer may or may not have air permeability and may be properly selected depending upon the utility. With respect to the air permeability, the adhesive layer may be air-permeable as a whole. Examples thereof include an adhesive layer having air permeability as a whole of a region in which an adhesive is partially present and a portion where no adhesive is present is partially present.

In laminating an adhesive on an air-permeable substrate and/or a covering material in a stratiform state as it is, examples of a method for keeping its air permeability include a method in which an adhesive layer is partially laminated by printing or transferring an adhesive, thereby forming a non-laminated part as an air-permeable part; a method in which an adhesive is transferred in one direction while drawing a circle in a filament-like form or properly moved in the two-dimensional directions by transferring in a zigzag manner, whereby a space of the filament-like adhesive keeps air permeability or moisture permeability or the adhesive is foamed; and a method for forming a layer by a melt blow system.

Examples of the adhesive which constitutes the non-hydrophilic adhesive layer include acrylic adhesives, polyvinyl acetate based adhesives (for example, vinyl acetate resin based emulsions and ethylene-vinyl acetate resin based holt melt adhesives), polyvinyl alcohol based adhesives, polyvinyl acetal based adhesives, vinyl chloride based adhesives, polyamide based adhesives, polyethylene based adhesives, cellulose based adhesives, chloroprene (neoprene) based adhesives, nitrile rubber based adhesives, polysulfide based adhesives, butyl rubber based adhesives, silicone rubber based adhesives, styrene based adhesives (for example, styrene based hot melt adhesives), rubber based adhesives, and silicone based adhesives. Of these, rubber based adhesives, acrylic adhesives, and adhesives containing a hot melt based polymer substance for the reasons that they are high in the adhesive strength, are cheap, are good in long-term stability, and are small in reduction of the adhesive strength even by providing heat.

In addition to the base polymer, if desired, the adhesive may be compounded with other components such as tackifiers (for example, petroleum resins represented by rosins, chroman-indene resins, hydrogenated petroleum resins, maleic anhydride-modified rosins, rosin derivatives, and C-5 based petroleum resins), phenol based tackifiers (especially, tackifiers having an aniline point of not higher than 50 °C; for example, terpene phenol based resins, rosin phenol based resins, and alkylphenol based resins), softeners (for example, coconut oil, castor oil, olive oil, camellia oil, and liquid paraffin), softeners, anti-aging agents, fillers, aggregates, adhesion adjusting agents, adhesion modifiers, coloring agents, anti-foaming agents, thickeners, and modifiers, thereby improving performance such as an improvement in adhesion to nylon-made clothes and mixed yarn clothes.

Examples of the hot melt based adhesive include known hot melt based adhesives imparted with adhesion. Specific examples thereof include styrene based adhesives made of, as a base polymer, an A-B-A type block copolymer (for example, SIS, SBS, SEBS, and SIPS), vinyl chloride based adhesives made of, as a base polymer, a vinyl chloride resin, polyester based adhesives made of, as a base polymer, a polyester, polyamide based adhesives made of, as a base polymer, a polyamide, acrylic adhesives made of, as a base polymer, an acrylic resin, polyolefin based adhesives made of, as a base polymer, a polyolefin (for example, polyethylene, super low density polyethylene, polypropylene, ethylene-α-olefin copolymers, and ethylene-vinyl acetate copolymers), 1,2-polybutadiene based adhesives made of, as a base polymer, 1, 2-polybutadiene, and polyurethane based adhesives made of, as a base polymer, polyurethane; adhesives made of a modified body of the foregoing adhesive whose adhesion is improved or whose stability is changed; and mixtures of two or more kinds of these adhesives. Adhesive layers constituted of a foamed adhesive and adhesive layers constituted of a crosslinked adhesive can also be employed.

The non-aromatic hot melt based adhesive is not limited so far as it is made of, as a base polymer, a hot melt based adhesive not containing an aromatic ring. Examples thereof include olefin based hot melt based adhesives and acrylic hot melt based adhesives. As the non-aromatic polymer which is the base polymer not containing an aromatic ring, there are enumerated polymers or copolymers of an olefin or a diene. Examples thereof include olefin polymers. The olefin polymer includes polymers or copolymers of ethylene or an α-olefin. Also, polymers resulting from adding a diene (for example, butadiene and isoprene) as other monomer thereto may be employed.

The α-olefin is not limited so far as it is a monomer having a double bond in the terminal thereof. Examples thereof include propylene, butene, heptane, hexene, and octene.

The "aromatic hot melt based adhesive" as referred to herein is a hot melt based adhesive whose base polymer contains an aromatic ring. Examples thereof include styrene based hot melt based adhesives represented by A-B-A type block copolymers.

In the foregoing A-B-A type block copolymers, the A block is a non-elastic polymer block made of a monovinyl substituted aromatic compound A such as styrene and methylstyrene; and the B block is an elastic polymer block made of a conjugated diene such as butadiene and isoprene. Specific examples thereof include a styrene-butadiene-styrene block copolymer (SBS), a styrene-isoprene-styrene block copolymer (SIS), and hydrogenated types thereof (for example, SEBS and SIPS), and mixtures thereof.

As a countermeasure for preventing a lowering of adhesive strength caused due to an increase of water of the non-hydrophilic adhesive layer, an adhesive layer obtained by further compounding a water absorptive polymer in the non-hydrophilic adhesive can be used.

The hydrophilic adhesive which constitutes the hydrophilic adhesive layer is not particularly limited so far as it contains a hydrophilic polymer or a water-soluble polymer as the major component, has adhesion and is hydrophilic as an adhesive.

Examples of the constitutional components of the hydrophilic adhesive include hydrophilic polymers (for example, polyacrylic acid), water-soluble polymers (for example, poly(sodium acrylate) and polyvinylpyrrolidone), crosslinking agents (for example, dry aluminum hydroxide and meta-silicic acid aluminic acid metal salts), softeners (for example, glycerin and propylene glycol), higher hydrocarbons (for example, soft liquid paraffin and polybutene), primary alcohol fatty acid esters (for example, isopropyl myristate), silicon-containing compounds (for example, silicone oil), fatty acid glycerin esters (for example monoglycerides), oily components (for example, vegetable oils such as olive oil), antiseptics (for example, methyl p-hydroxybenzoate and propyl p-hydroxybenzoate), solubilizing agents (for example, N-methyl-2-pyrrolidone), thickeners (for example, carboxymethyl cellulose), surfactants (for example, polyoxyethylene hardened castor oil and sorbitan fatty acid esters), hydroxycarboxylic acid (for example, tartaric acid), excipients (for example, light silicic anhydride, water absorptive polymers, and kaolin), moisturizers (for example, D-sorbitol), stabilizers (for example, sodium edetate, p-hydroxybenzoic acid esters, and tartaric acid), crosslinking type water absorptive polymers, boron compounds (for example, boric acid), and water. They may be used as an arbitrary combination.

A temporary adhering seal part is formed via a sticky layer. An adhesive which constitutes the sticky layer is a layer formed of a polymer composition which is tacky at the normal temperature and is not limited so far as it can be heat sealed after temporary adhesion.

Furthermore, the foregoing adhesives of the sticky layer can be used as the adhesive which constitutes the sticky layer as used for temporary adhesion. Of these, non-hydrophilic adhesives are preferable. With respect to the adhesive constituting the adhesive layer, it is preferable that the adhesive is well compatible with a heat seal material constituting a heat seal and that a melting point of the base polymer of the adhesive is not higher than a melting point of the heat seal material. Hot melt based adhesives are especially preferable for hot melt based bonding agents. Furthermore, in the case where the heat seal material is an olefin based raw material, preferred examples thereof include olefin based adhesives.

A bonding layer for fixing the air permeability adjusting material is constituted of a bonding agent or an adhesive which is usually used. In particular, an adhesive is useful, and the foregoing adhesives for constituting the adhesive layer can be used.

Furthermore, a method for providing a bonding layer is not limited so far as the air permeability adjusting material can be fixed. The bonding layer may be entirely provided or partially or intermittently provided. Examples of its shape include various shapes such as a network-like shape, a stripe-like shape, a dot-like shape, and strip-like shape.

Furthermore, in the case where an adhesive layer is employed as the hydrophilic adhesive layer, if there is a difference in a water retaining force between the hydrophilic adhesive layer and the heat generating composition molded body, transfer of water occurs via a packaging material present therebetween such as a substrate, thereby causing in-conveniences against the both. In particular, the transfer of water occurs during the storage. In order to prevent this, it is preferable that the packaging material present therebetween at least has a moisture permeability of not more than 2 $g/m^2/day$ in terms of a moisture permeability according to the Lyssy method. By using this, in the case where the heat generating body is accommodated in an outer bag as an air-impermeable accommodating bag and stored, the transfer of water can be prevented.

In the case where a hydrophilic adhesive layer is used as the adhesive layer, the moisture permeability of a moisture-proof packaging material provided between the heat generating composition molded body and the hydrophilic adhesive layer is not limited so far as the transfer of water can be prevented within the range where the exothermic performance is not affected. The moisture permeability according to the Lyssy method is usually not more than 2 $g/m^2/day$, preferably not more than 1.0 $g/m^2/day$, more preferably not more than 0.5 $g/m^2/day$, and further preferably from 0.01 to 0.5 $g/m^2/day$. These values are a value under a condition under an atmospheric pressure at 40 °C and 90 % RH. Incidentally, the moisture-proof packaging material can be used as a substrate or a covering material and may be laminated singly on a substrate, a covering material, or the like.

The moisture-proof packaging material is not limited so far as the transfer of water between the heat generating composition molded body and the hydrophilic adhesive layer can be prevented. Examples thereof include metal vapor deposited films, vapor deposited films of a metal oxide, metal foil-laminated films, EVOH (ethylene/vinyl alcohol copolymer or ethylene/vinyl acetate copolymer saponified product) based films, biaxially stretched polyvinyl alcohol films, polyvinylidene chloride coated films, polyvinylidene chloride coated films obtained by coating polyvinylidene chloride on a substrate film (for example, polypropylene), metal foils such as an aluminum foil, air-impermeable packaging materials obtained by vapor depositing or sputtering a metal (for example, aluminum) on a polyester film substrate, and packaging laminates using a transparent barrier film of a structure in which silicon oxide or aluminum oxide is provided on a flexible plastic substrate. The air-impermeable packaging materials which are used in the outer bag, etc. can also be used.

Furthermore, packaging materials such as moisture-proof packaging materials as described in JP-A-2002-200108, the disclosures of which can be incorporated herein by reference, can be used.

In the case of using a water-containing hydrophilic adhesive (for example, a gel) in the adhesive layer, in order to adjust the moisture equilibrium between the heat generating composition and the adhesive layer, the content of a reaction accelerator (for example, sodium chloride) or a substance having a water holding power (for example, a water absorptive polymer) in the heat generating composition may be adjusted within the range of from 10 to 40 % by weight, preferably from 15 to 40 % by weight, and more preferably from 15 to 30 % by weight based on the heat generating composition. Furthermore, as the adhesive having good moisture permeability and low stimulation to the skin, water-containing adhesives (for example, hydrophilic adhesives and gels) as described in JP-A-10-265373 and JP-A-9-87173, adhesives which can be subjected to hot melt coating as described in JP-A-6-145050 and JP-A-6-199660, and rubber based adhesives as described JP-A-10-279466 and JP-A-10-182408, the disclosures of which are totally incorporated herein by reference, are useful.

The functional substance which is contained in the adhesive layer is not limited so far as it is a substance having any function. There can be enumerated at least one member selected from aromatic compounds, vegetable extracts, crude drugs, perfumes, slimming agents, analgesics, blood circulation promoters, swelling improvers, antibacterial agents, sterilizers, mold inhibitors, odor eaters, deodorants, percutaneously absorptive drugs, fat-splitting components, minus ion generators, far infrared ray radiants, magnetic bodies, fomentations, cosmetics, bamboo vinegar, and wood vinegar. Specific examples thereof include aromatic compounds (for example, menthol and benzaldehyde), vegetable extracts (for example, mugwort extract), crude drugs (for example, moxa), perfumes (for example, lavender and rosemary), slimming agents (for example, aminophylline and tea extract), analgesic drugs (for example, indomethacin and *dl*-camphor), blood circulation promoters (for example, acidic mucopolysaccharide and chamomile), swelling improvers (for example, horse chestnut extract and flavone derivatives), fomentations (for example, aqueous boric acid, physiological saline, and aqueous alcohols), fat-splitting components (for example, jujube extract, caffeine, and tonalin), cosmetics (for example, aloe extracts, vitamin preparations, hormone preparations, anti-histamines, and amino acids), antibacterial agents and sterilizers (for example, carbolic acid derivatives, boric acid, iodine preparations, invert soaps, salicylic acid based substances, sulfur, and antibiotics), and mold inhibitors.

The percutaneously absorptive drug is not particularly limited so far as it has percutaneous absorption. Examples thereof include corticosteroids, anti-inflammatory drugs, hypertension drugs, anesthetics, hypnotic sedatives, tranquillizers, antibacterial substances, antifungal substances, skin stimulants, inflammation inhibitors, anti-epileptics, analgesics, antipyretics, anesthetics, mold inhibitors, antimicrobial antibiotics, vitamins, antiviral agents, swelling improvers, diuretics, antihypertensives, coronary vasodilators, anti-tussive expectorants, slimming agents, anti-histamines, antiarrhythmic agents, cardiotonics, adrenocortical hormones, blood circulation promoters, local anesthetics, fat-splitting components, and mixtures thereof. However, it should not be construed that the invention is limited thereto. These drugs are used singly or in admixture of two or more kinds thereof as the need arises.

The content of such a functional substance is not particularly limited so far as it falls within the range where the effect of a medicine can be expected. However, from the viewpoints of adhesive strength as well as pharmacological effect and economy, the content of the functional substance is preferably from 0.01 to 25 parts by weight, and more preferably from 0.5 to 15 parts by weight based on 100 parts by weight of the adhesive.

Furthermore, a method for providing the adhesive layer is not limited so far as a thermal packaging body for joint surroundings can be fixed. The adhesive layer may be entirely provided or partially or intermittently provided. Examples of its shape include various shapes such as a network-like shape, a stripe-like shape, a dot-like shape, and strip-like shape.

At least one member or a part of the substrate, the covering material, the adhesive layer and the separator which constitute the heat generating body may be provided with at least one kind of characters, designs, symbols, numerals, patterns, photographs, pictures, and colored parts.

Each of the substrate, the covering material, the adhesive layer and the separator which constitute the heat generating body may be transparent, opaque, colored, non-colored, or the like. Furthermore, the layer constituting at least one layer of the layers constituting the respective materials and layers may have a colored part as colored different from other layers.

The heat generating body of the invention is able to give various shapes, thicknesses and temperature zones and therefore, can be used for various utilities such as use for indirect moxibustion, use for inside of shoes for feet, etc., use for a joint, facial esthetic use, use for eyes, use for a wet compress pack, use for a medical body warmer, use for a neck, use for a waist, use for a mask, use for a glove, use for hemorrhage, use for a shoulder, use for a cushion, use for an aroma, use for an abdomen, insecticidal use by thermal volatilization, use for oxygen absorption, and use for treating cancer in addition to common warming of a human body. In addition, the heat generating body of the invention can be used for heat insulation of pets, machines, and the like.

Examples of a method for using the heat generating body include a method of use in which the heat generating body is applied to a site of the body which a person who requires the remedy has a pain, the temperature of the skin or the holding time is appropriately selected depending upon the person who requires the remedy, and a suffering is substantially relieved in comfort, thereby remedying the suffering due to an acute, recurrent or chronic muscular pain, a skeleton pain, or a related pain.

Although the process for producing the heat generating body is not limited, there are enumerated the following production processes.

1) Filling system:

The filling system is a method for coupling an end of a substrate or a partition part by an adhesive, sewing processing, or an appropriate system such a heat seal system to form a bag, filling a heat generating composition in the bag, and then bonding the end of the bag. As a process for producing a compartmentalized heat generating body by the filling system, there is enumerated a continuous formation method in which by using a longitudinal substrate and a rotary heat compressioning unit capable of heat sealing a desired partition part and the periphery of the substrate, the periphery of the longitudinal substrate and a necessary part of the partitioned as disposed opposite to each other via the heat compression unit are heated sealed, and at the same time, an air-permeable heat generating body is fed into a compartment formed of space between the substrates and subjected to a seal treatment, and the formation of a next compartment is started while bonding an end of the body warmer by this seal treatment.

2) Pocket system:

As disclosed in JP-T-11-508786, the pocket system is a process for producing a heat generating body in which a pocket is prepared in advance on a substrate by thermal molding, mechanical embossing, vacuum embossing, or other tolerable means, a heat generating composition and its compressed body, etc. are filled in the pocket, the pocket is covered by another substrate, and the surroundings of the two substrates are coupled.

3) Molding system:

The molding system is a process for producing a heat generating body in which a moldable heat generating composition is molded into a desired shape by a force-through molding method using a trimming die or a cast molding method using a casting mold, the molded body is laminated on a substantially planar substrate not having an accommodating pocket, etc., and another substrate is covered thereon, followed by sealing.

The "force-through molding method" as referred to herein means a continuous formation method in which by using a molding machine for laminating a heat generating composition molded body having a trimming die shape on a longitudinal substrate by using a trimming die and a rotary seal unit capable of covering the laminate by a longitudinal covering material and sealing (by heat seal, compression seal, or heat compression seal) a desired sectioned part and the surroundings of the substrate and the covering material, the surroundings of the heat generating composition molded body and a necessary part of the sectioned part are heat sealed via the seal unit and subjected to a seal treatment.

Furthermore, the "cast molding method" as referred to herein means a molding method for laminating a heat generating composition molded body on a longitudinal substance by filling in a casting mold having a concave and transferring it into a substrate.

In the continuous case, there is enumerated a continuous formation method in which by using a molding machine for laminating a heat generating molding molded body on a longitudinal substrate by filling in a concave and transferring into a substrate by a drum-type body of rotation and a rotary seal unit capable of covering the laminate by a longitudinal covering material and sealing (by heat seal, compression seal, or heat compression seal) a desired sectioned part and the surroundings of the substrate and the covering material, the surroundings of the heat generating composition molded body and a necessary part of the sectioned part are heat sealed via the seal unit and subjected to a seal treatment.

Furthermore, in producing a heat generating body using the heat generating composition of the invention according to the foregoing methods or other methods, a magnet may be used. By using a magnet, it becomes possible to easily achieve accommodation of the heat generating composition in a bag or a mold and separation of the molded body from the mold, thereby making it easier to mold a heat generating composition molded body or produce a heat generating body.

The "air-permeable adjusting material" as referred to in the invention comprises a sectional exothermic part and a sectioned part and covers an exothermic part having a difference of altitude via an adhesive layer, etc., thereby adjusting the air permeability into the sectional exothermic part. That is, in the air permeability adjusting material, by covering the exothermic part by the air-permeable adjusting material while utilizing a difference of altitude between the sectional exothermic part and the sectioned part, a partitioned space is formed in at least a part of the periphery of the sectional exothermic part, thereby adjusting the air permeability between the outside and the sectional exothermic part and also imparting a heat insulating effect.

The air permeability of the air permeability adjusting material is not limited so far as it is able to adjust air retention or air permeability in at least a part of the periphery of the sectional exothermic part. However, it is preferable that the air permeability of the air permeability adjusting material is lower than that on the air-permeable surface of the sectional exothermic part as a covering part for covering the heat generating composition molded body.

Furthermore, a region where the air permeability is higher than that in the covering part for covering the heat generating composition molded body may be provided in a local region of the air-permeable adjusting material, thereby keeping the air permeability of other region lower than that on the air-permeable surface of the sectional exothermic part. In this way, it is possible to control an air passage for air, etc.

As the raw material which constitutes the air permeability adjusting material, the raw material which is used in the packaging material to be used in the air-impermeable accommodating bag for sealing and accommodating the substrate, the covering material and the heat generating body of a chemical body warmer or heat generating body can be used. An adhesive which is used in a chemical body warmer or heat generating body is preferable.

The heat generating body of the invention is able to give various shapes, thicknesses and temperature zones and therefore, can be used for various utilities such as use for a joint, facial esthetic use, use for eyes, slimming use, use for heating or warming a dripping solution, use for a wet compress pack, use for a medical body warmer, use for a neck, use for a waist, use for a mask, use for a glove, use for hemorrhage, use for relaxation of symptoms such as shoulder pain, muscular pain, and menstrual pain, use for a cushion, use for heating or warming a human body during the operation, use for a thermal sheet, use for thermally volatilizing an aroma, use for an abdomen, insecticidal use by thermal volatilization, and use for treating cancer in addition to common warming of a human body. In addition, the heat generating body of the invention can be used for heating or warming machines, pets, etc.

For example, in the case of using for relaxation of symptoms, the heat generating body of the invention is applied directly in a necessary site of the body or indirectly via a cloth, etc. Furthermore, in the case of using for heating or warming a human body during the operation, a method for using the heat generating body of the invention includes the following methods.

(1) The heat generating body is directly applied to a body requiring heating or warming.
(2) The heat generating body is fixed on a covering, etc. and covered on the body.
(3) The heat generating body is fixed on a cushion to be placed beneath the body, etc.
(4) The heat generating body is used as a covering or a cushion which is a product having the heat generating body provided therein in advance.

Incidentally, examples of the pain of muscles or bones include acute muscle pain, acute bone pain, acute reference pain, previous muscle pain, previous bone pain, chronic reference pain, and join pain of knee, elbow, etc.

The holding time is not limited but is preferably from 20 seconds to 24 hours, more preferably from one hour to 24 hours, and further preferably from 8 hours to 24 hours.

The holding temperature is preferably from 30 to 50 °C, more preferably from 32 to 50 °C, further preferably from 32 to 43 °C, still further preferably from 32 to 41 °C, and even further preferably from 32 to 39 °C.

The invention will be specifically described below with reference to the Examples, but it should not be construed that the invention is limited thereto.

[Brief Description of the Drawings]

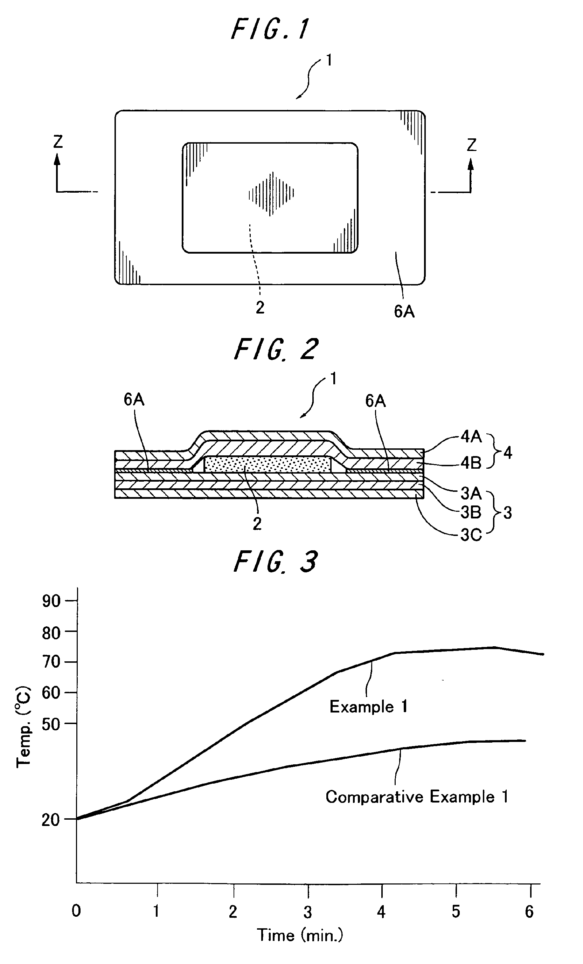

[Fig. 1] is a plan view of an embodiment of the heat generating body of the invention.
[Fig. 2] is a cross-sectional view along the line Z-Z of the same.
[Fig. 3] is a diagram of exothermic characteristics of the heat generating composition of the invention.

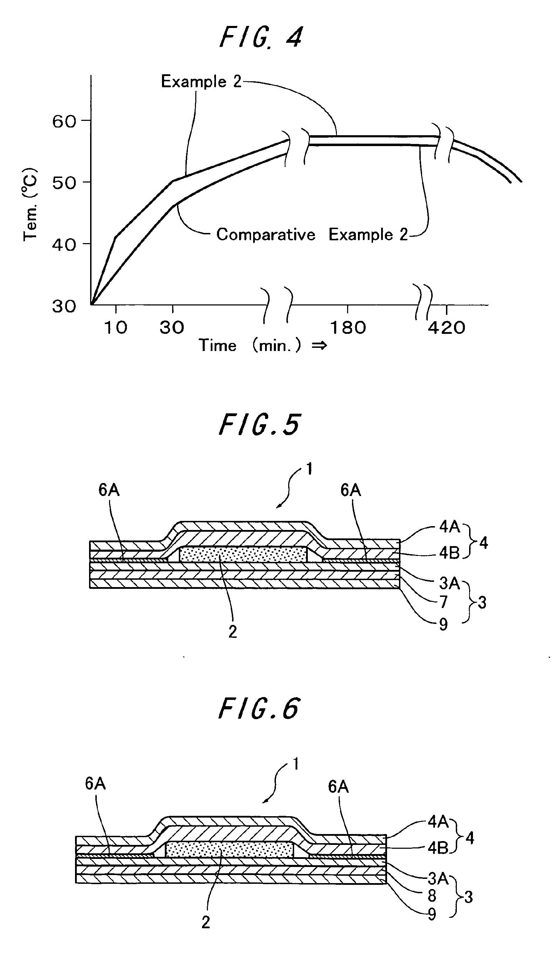

[Fig. 4] is a diagram of exothermic characteristics of the heat generating body of the invention.
[Fig. 5] is a cross-sectional view of another embodiment of the heat generating body of the invention.
[Fig. 6] is a cross-sectional view of a still another embodiment of the heat generating body of the invention.

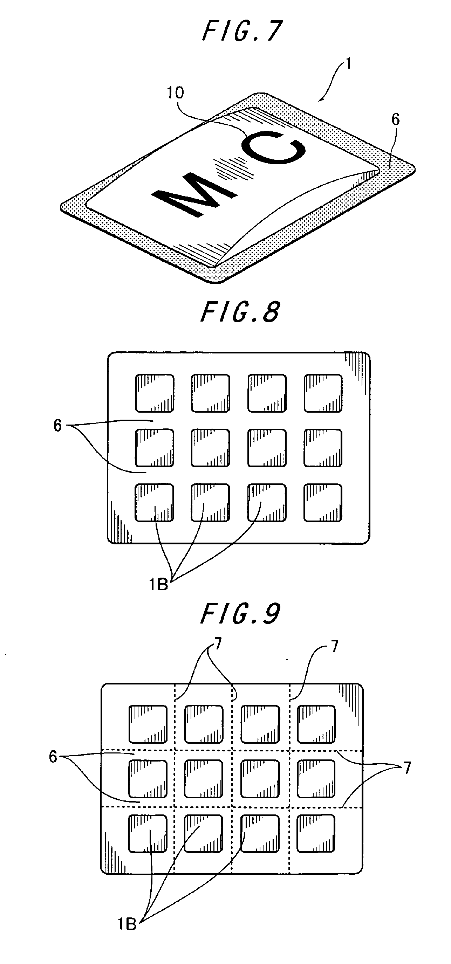

[Fig. 7] is an oblique view of another embodiment of the heat generating body of the invention.
[Fig. 8] is a plan view of another embodiment of the heat generating body of the invention.
[Fig. 9] is a plan view of a still another embodiment of the heat generating body of the invention.

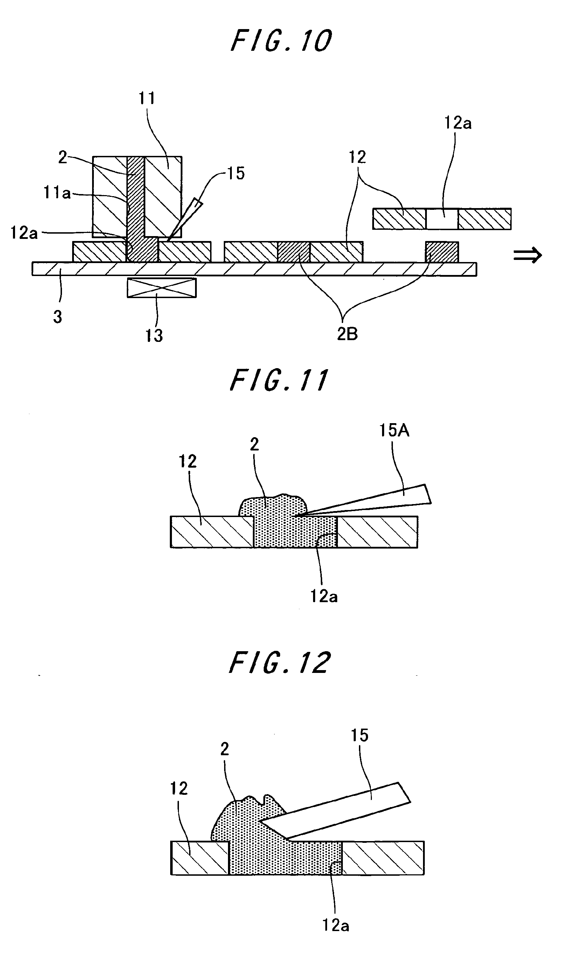

[Fig. 10] is a schematic view of force-through molding of the heat generating body of the invention using a leveling plate.
[Fig. 11] is an explanatory view in the vicinity of the leveling plate of the same.
[Fig. 12] is a schematic view of force-through molding of the heat generating body of the invention using a pushing leveling plate.
[Fig. 13] is a plan view of a filter paper for the measurement of water mobility value in the invention.
[Fig. 14] is an oblique view for explaining the measurement of water mobility value in the invention.
[Fig. 15] is a cross-sectional view of the same.
[Fig. 16] is a cross-sectional view of the same.
[Fig. 17] is a plan view of a filter paper after carrying out the measurement of water mobility value in the invention.

[Description of Reference Numerals and Signs]

1:       Heat generating body
1B:     Sectional exothermic part
2:       Heat generating composition molded body
3:       Substrate
3A:     Polyethylene film
3B:     Non-woven fabric
4:       Covering material
4A:     Porous film
4B:     Non-woven fabric
6:       Sectioned part
7:       Perforation
8:       Adhesive layer

8A:     Hydrophilic adhesive layer
9:      Separator
10:     Design
11:     Die
11a:    Die hole
12:     Mold
12a:    Mold hole
13:     Magnet
14:     Pushing plate
15:     Leveling plate
15A:    Pushing leveling plate
16:     Flat plate
17:     Non-water absorptive film (polyethylene film, etc.)
18:     Filter paper in which eight lines are drawn radiating from
the     central point with an interval of 45°
19:     Die plate
20:     Hole
21:     Sample
22:     Stainless steel plate
23:     Distance to the oozed-out locus of water or aqueous solution
24:     Position corresponding to a hollow cylindrical hole on filter paper

[Examples]

(Example 1)

A stirring type batchwise oxidizing gas contact treatment device consisting of a mixer equipped with a rotary blade for stirring was used as an oxidizing gas contact treatment device, and air was used as an oxidizing gas.
First of all, a reaction mixture consisting of 100 parts by weight of a reduced iron powder (particle size: not more than 300 $\mu$m), 3.5 parts by weight of active carbon (particle size: not more than 300 $\mu$m), and 5 parts by weight of 11 % salt water and having a water mobility value of less than 0.01 was charged in the stirring type batchwise oxidizing gas contact treatment device. Next, the upper portion of the oxidizing gas contact treatment device was opened to air, the reaction mixture was subjected to self heat generation with stirring under circumstances at 25 °C, and at a point of time when the temperature reached 30 °C after 20 seconds, the reaction mixture was sealed in an air-impermeable accommodating bag and allowed to stand until the temperature reached room temperature, thereby obtaining a heat generating mixture of the invention.
Next, 11 % salt water was added to and mixed with the heat generating mixture to obtain a heat generating composition having a water mobility value of 8.

(Comparative Example 1)

A reaction mixture the same as in Example 1 was not subjected to the oxidizing gas contact treatment, and 11 % by weight salt water was added to the reaction mixture, thereby obtaining a heat generating composition having a water mobility value of 8. Using the heat generating composition, a heat generating body was obtained in the same manner as in Example 1.
The heat generating compositions of Example 1 and Comparative Example 1 were each subjected to an exothermic test of heat generating composition.
As a result, as seen in the curves of Fig. 3, the heat generating composition of Example 1 reached about 35 °C after one minute and about 55 °C (an average value of five samples) after three minutes, respectively.
In the heat generating composition of Comparative Example 1, the temperature was 23 °C after one minute and 28 °C (an average value of five samples) after three minutes, respectively. Comparative Example 1 was remarkably slow with respect to the temperature rise rate as compared with Example 1.
From the test results of Example 1 and Comparative Example 1, it could be confirmed that the heat generating composition as prepared in Example 1 is excellent with respect to the exothermic rising properties.

(Example 2)

A stirring type batchwise oxidizing gas contact treatment device consisting of a mixer equipped with a rotary blade for stirring was used as an oxidizing gas contact treatment device, and air was used as an oxidizing gas.

A reaction mixture consisting of 100 parts by weight of an iron powder (particle size: not more than 300 $\mu$m), 5. 5 parts by weight of active carbon (particle size: not more than 300 $\mu$m), 2.3 parts by weight of a wood meal (particle size: not more than 150 $\mu$m), 2.3 parts by weight of a water absorptive polymer (particle size: not more than 300 $\mu$m), 0.5 parts by weight of calcium hydroxide, 0.7 parts by weight of sodium sulfite, and 10 parts by weight of 11 % salt water and having a water mobility value of less than 0.01 was charged in the stirring type batchwise oxidizing gas contact treatment device.

Next, the upper portion of the oxidizing gas contact treatment device was opened to air, the reaction mixture was subjected to self heat generation with stirring under circumstances at 20 °C, and at a point of time when the temperature reached 27 °C after 20 seconds, the reaction mixture was sealed in an air-impermeable accommodating bag and allowed to stand until the temperature reached room temperature, thereby obtaining a heat generating mixture of the invention.

Next, 11 % salt water was added to and mixed with the heat generating mixture to obtain a heat generating composition having a water mobility value of 8.

This heat generating composition was subjected to the exothermic test of heat generating composition. As a result, the same results as in Example 1 were obtained.

Furthermore, the heat generating composition was tested for moldability. As a result, even after separating a trimming die from a heat generating composition molded body, the heat generating composition molded body was free from a loss of shape, and collapsed pieces of the heat generating composition molded body were not generated in the surroundings of the heat generating composition molded body.

Next, as shown in Figs. 1 and 2, an air-permeable substrate 3 having an adhesive layer 3B and a separator 3C provided on a polyethylene film 3A was used, and a rectangular heat generating composition molded body 2 having a plane of 2 mm in thickness, 115 mm in length and 80 mm in width was molded on the side of the polyethylene film 3A using a trimming die having a thickness of 2 mm and laminated on the substrate 3. In addition, an air-permeable packaging material of a laminate of a nylon-made non-woven fabric 4A and a polyethylene-made porous film 4B was used as a covering material 4 and superimposed thereon such that the surface of the polyethylene film 3A and the surface of the porous film 4B were brought into contact with each other, and the periphery of the heat generating composition molded body was subjected to a heat seal 6A in a seal width of 8 mm, thereby preparing an irregular heat generating body 1 having a rectangular shape of 135 mm in length, 100 mm in width and 8 mm in seal width.

Incidentally, the air permeability of the covering material 4 was 370 g/m$^2$/24 hr in terms of a moisture permeability by the Lyssy method.

The heat generating body was sealed and accommodated in an air-impermeable outer bag and allowed to stand at room temperature for 24 hours. An exothermic test by the body was carried out. As a result, it was felt warm after 3 minutes, and thereafter, the warmth was continued for 10 hours or more.

(Comparative Example 2)

A reaction mixture the same as in Example 2 was not subjected to the oxidizing gas contact treatment, and salt water of 11 % by weight was added to the reaction mixture, thereby obtaining a heat generating composition having a water mobility value of 8. As a result of the exothermic test of heat generating composition, the same results as in Comparative Example 1 were obtained. Furthermore, using the heat generating composition, a heat generating body was obtained in the same manner as in Example 2.

With respect to Example 2 and Comparative Example 2, the exothermic test of heat generating body was carried out. As a result, as shown in Fig. 4, in the case of Example 2, the temperature was 40 °C after 10 minutes, 50 °C after 30 minutes and 58 °C after 3 hours, respectively. However, in the case of Comparative Example 2, the temperature was 35 °C after 10 minutes, 45 °C after 30 minutes and 55 °C after 3 hours, respectively. The heat generating body using the heat generating composition of the invention was explicitly excellent with respect to the exothermic rising properties.

Fig. 5 is a cross-sectional view of the heat generating body 1 in which the substrate 3 of Example 2 is replaced by a substrate 3 having an SIS based adhesive layer 7 equipped with a separator 9.

Fig. 6 is a cross-sectional view of the heat generating body 1 in which the substrate of Example 2 is replaced by a hydrophilic adhesive layer 8 equipped with a separator 9. A hydrophilic adhesive constituting the hydrophilic adhesive layer is a composition consisting of 4.5 % by weight of polyacrylic acid, 1.5 % by weight of poly(sodium acrylate), 4.0 parts by weight of sodium carboxymethyl cellulose, 15.0 % by weight of glycerin, 5.0 % by weight of propylene glycol, 10.0 % by weight of sorbitol, 0.1 % by weight of aluminum hydroxide, 0.05 % by weight of synthetic hydrotalcite, and 1. 0 % by weight of polyoxyethylene glycol, with the remainder being water.

Fig. 7 is an example in which a display 10 is provided in the heat generating body 1.

(Example 3)

To the heat generating mixture as obtained in Example 1, a mixture consisting of 100 parts by weight of an iron powder, 2.3 parts by weight of a water absorptive polymer (particle size: not more than 300 $\mu$m), 2. 3 parts by weight of a wood meal (particle size: not more than 300 $\mu$m), 0.7 parts by weight of sodium sulfite, and 0.2 parts by weight of calcium hydroxide (particle size: not more than 300 $\mu$m) as reduced into the reaction mixture was added. After mixing, 30 parts by weight of 11 % salt water was added and further mixed, thereby obtaining a heat generating composition. Its water mobility value was less than 0.01.

This heat generating composition was subjected to an exothermic test of heat generating composition to obtain 5 data in total.

The results of measuring the temperature revealed that the temperature was about 50 °C (an average value of five samples) after 3 minutes likewise Example 1.

Furthermore, the heat generating composition was tested for moldability. As a result, even after separating a trimming die from a heat generating composition molded body, the heat generating composition molded body was free from a loss of shape, and collapsed pieces of the heat generating composition molded body were not generated in the surroundings of the heat generating composition molded body.

Next, as explained using the foregoing Fig. 6, packing material pieces of 135 mm in length x 100 mm in width were prepared by using an air-impermeable packaging material having an adhesive layer 8 and a separator 9 on a polyethylene film 3A as a substrate 3 and an air-permeable packaging material having a nylon-made non-woven fabric 4A and a porous polyethylene film 4B laminated in this order as a covering material 4, respectively, and a rectangular air-permeable flat accommodating bag, three sides of which were heat sealed in a seal width of 8 mm, was prepared. 25 g of the foregoing heat generating composition was charged in the flat accommodating bag, and the side which had not been heat sealed was heat sealed, thereby preparing a rectangular flat heat generating body 1 of 135 mm in length, 100 mm in width and 8 mm in seal width. Incidentally, the air permeability of the covering material 4 was 370 g/m$^2$/24 hr in terms of a moisture permeability by the Lyssy method. The heat generating body was sealed and accommodated in an air-impermeable outer bag and allowed to stand at room temperature for 24 hours.

After 24 hours, the heat generating body was taken out from the outer bag and subjected to an exothermic test. The temperature after lapsing 30 minutes was 50 °C. A temperature of 40 °C or higher was continued for 10 hours or more.

(Example 4)

A heat generating composition was obtained in the same manner as in Example 3, except that the water mobility value was changed to 5.

This heat generating composition was subjected to an exothermic test of heat generating composition.

The measurement results revealed that the temperature after 3 minutes was about 49 °C (an average value of five samples).

By using the same substrate and covering material as in Example 3, a rectangular heat generating composition molded body of 125 mm in length x 90 mm in width was molded and laminated on the substrate by force-through molding. Next, the air-permeable covering material was covered thereon, and the surroundings of the heat generating composition molded body were heat sealed, thereby preparing a rectangular heat generating body of 135 mm in length x 100 mm in width in a seal width of 8 mm.

The heat generating body was sealed and accommodated in an air-impermeable outer bag and allowed to stand at room temperature for 24 hours. After 24 hours, the heat generating body was taken out from the outer bag and subjected to an exothermic test.

The temperature after 30 minutes was 52 °C. A temperature of 40 °C or higher was continued for 10 hours or more.

(Example 5)

25 g of the heat generating composition as prepared in Example 3 was accommodated in a separator-protected adhesive layer-provided air-permeable accommodating bag, and the surroundings of the heat generating body were sealed in a seal width of 8 mm to prepare a rectangular flat heat generating body of 130 mm in length x 80 mm in width, which was then sealed in an air-impermeable outer bag. The air permeability of an air-permeable part of the air-permeable accommodating bag was 400 g/m$^2$/24 hr in terms of a moisture permeability by the Lyssy method.

After 24 hours, the heat generating body was taken out from the outer bag and subjected to an exothermic test by the body. As a result, it was felt warm within 3 minutes, and thereafter, the warmth was continued for 10 hours or more.

(Example 6)

A reaction mixture consisting of 100 parts by weight of an iron powder (particle size: not more than 300 μm), 6.5 parts by weight of active carbon (particle size: not more than 300 μm), 3 parts by weight of a water absorptive polymer (particle size: not more than 300 μm), 0.5 parts by weight of calcium hydroxide, 0.7 parts by weight of sodium sulfite, and 10 parts by weight of 11 % salt water and having a water mobility value of less than 0.01 was charged in a stirring type batchwise oxidizing gas contact treatment device under circumstances at 30 °C. The temperature of the reaction mixture at the time of charging was 20 °C.

Next, the upper portion of the oxidizing gas contact treatment device vessel was opened to air, and at a point of time when the temperature reached 40 °C after one minute, 11 % salt water was added with stirring. Stirring and mixing were carried out for 10 seconds to form a heat generating composition having a water mobility value of 10. A heat generating body was prepared in the same manner as in Example 5 and subjected to an exothermic test. As a result, the same results as in Example 5 were obtained.

Furthermore, the heat generating composition was tested for moldability. As a result, even after separating a trimming die from a heat generating composition molded body, the heat generating composition molded body was free from a loss of shape, and collapsed pieces of the heat generating composition molded body were not generated in the periphery of the heat generating composition molded body.

(Example 7)

A stirring type batchwise oxidizing gas contact treatment device consisting of a mixer equipped with a rotary blade for stirring was used as an oxidizing gas contact treatment device, and air was used as an oxidizing gas.

A heat generating mixture consisting of 100 parts by weight of an iron powder (particle size: not more than 300 μm) having a content of wustite of less than 1 % by weight, 2.3 parts by weight of a wood meal (particle size: not more than 150 μm), 6.5 parts by weight of active carbon (particle size: not more than 300 μm), 2. 3 parts by weight of a water absorptive polymer (particle size: not more than 300 μm), 0.5 parts by weight of calcium hydroxide, 0.7 parts by weight of sodium sulfite, and 10 parts by weight of 8 % salt water and having a water mobility value of less than 0.01 was charged in the contact treatment device vessel.

Next, the upper portion of the contact treatment device vessel was opened to air, the reaction mixture was subjected to self heat generation with stirring under circumstances at 20 °C, and at a point of time when the temperature reached 50 °C after 3 minutes, 8 % salt water was added, and the mixture was stirred for 15 seconds, thereby obtaining a heat generating composition having a water mobility value of 8.

11 % salt water was added to the heat generating mixture for oxidizing gas contact treatment to adjust the water content and then mixed to obtain a heat generating composition having a water mobility value of 8. Next, as explained using the foregoing Fig. 6, an air-impermeable packaging material having an adhesive layer 8 and a separator 9 provided on a polyethylene film 3A was used as a substrate 3, and 12 square heat generating composition molded bodies having a thickness of 2 mm and a side length of 25 mm were laminated on the substrate in the polyethylene film side by force-through molding using a trimming die having a thickness of 2 mm and containing 12 square cavities having a side length of 15 mm. In addition, an air-permeable packaging material made of a laminate of a nylon-made non-woven fabric 4A and a polyethylene-made porous film 4B was used as a covering material 4 and superimposed thereon such that the surface of the polyethylene film and the surface of the porous film were brought into contact with each other. The surroundings of each of the square heat generating composition molded bodies were then heat sealed in a seal width of 5 mm, thereby preparing a sectioned part 6. In addition, the surroundings of the heat generating body were heat sealed in a seal width of 8 mm to prepare a heat generating body 1 having an outer dimension of 131 mm x 101 mm and containing 12 sectional exothermic parts 1B (see Fig. 8).

Furthermore, even after separating a trimming die from a heat generating composition molded body, the heat generating composition molded body was free from a loss of shape, and collapsed pieces of the heat generating composition molded body were not generated in the periphery of the heat generating composition molded body. Also, sealing could be completely carried out without causing incorporation of collapsed pieces of the heat generating composition molded body into the seal part, and seal failure did not occur. The moldability was satisfactory.

Incidentally, the air permeability of the covering material 4 was 370 g/m$^2$/24 hr in terms of a moisture permeability by the Lyssy method.

The heat generating body 1 was sealed and accommodated in an air-impermeable outer bag and allowed to stand at room temperature for 24 hours. After 24 hours, the heat generating body was taken out from the outer bag and subjected to an exothermic test by the body. As a result, it was felt warm within 3 minutes, and an exothermic holding time of 34 °C or higher was 8.5 hours. Flexibility of the heat generating body was kept before, during and after the use. An adhesive strength of the adhesive layer beneath of the sectioned part 6 of this heat generating body is weaker than that of the adhesive layer beneath the sectional exothermic part 1B.

Fig. 9 is an example in which a seal part 6 is sealed in an irregular pattern, and perforations 7 which can be cut by hand are provided.

(Example 8)

A heat generating composition having a water mobility value of 25 was obtained in the same manner as in Example 3, except for changing only the adjustment of the water content. Furthermore, the moldability of the heat generating composition was measured according to the foregoing measurement method for moldability. As a result, collapsed pieces of a heat generating composition molded body were not observed, and therefore, this heat generating composition was a moldable heat generating composition.

Next, an air-impermeable packaging material having a 40 $\mu$m-thick air-impermeable polyethylene film stuck on one surface of a liner paper (thickness: 300 $\mu$m) was used as a substrate, and the foregoing heat generating composition was molded and laminated on the liner paper of the substrate by force-through molding using a trimming die having a rectangular cavity of 2 mm in thickness, 120 mm in length and 84 mm in width, thereby obtaining a heat generating composition molded body. Collapsed pieces of a heat generating composition molded body were not observed in the periphery of the heat generating composition molded body, and the moldability was satisfactory.

In addition, a covering material in which a network-like adhesive layer provided with a styrene-isobutene-styrene block copolymer based sticky polymer in a network form by the melt blow method was provided in the side of a polyethylene-made porous film was superimposed thereon such that the network-like adhesive layer surface of the covering material and the heat generating composition molded body were brought into contact with each other. The periphery of the heat generating composition molded body was sealed by compression seal, and the circumference was cut, thereby producing a rectangular flat heat generating body of 136 mm in length, 100 mm in width and 8 mm in seal width. The heat generating body was sealed in an outer bag which is an air-impermeable accommodating bag and then allowed to stand for 24 hours. Here, a laminate of a craft paper having a thickness of 30 $\mu$m, a polyethylene-made porous film having a thickness of 50 $\mu$m, and a nylon non-woven fabric having a thickness of 150 $\mu$m in this order was used as the covering material. The network-like adhesive layer was provided on the craft paper. Incidentally, the air permeability of the covering material was 650 g/m$^2$/24 hr in terms of a moisture permeability by the Lyssy method.

Furthermore, after 24 hours, the heat generating body was taken out from the outer bag and subjected to an exothermic test by the body. As a result, it was felt warm within 3 minutes, and a period of time when it was felt warm was so long as 8 hours.

(Example 9)

Fig. 10 shows an embodiment of the force-through molding method using a leveling plate 15.

That is, a substrate 3 in a roll film form having a width of 130 mm is adapted to a molding mold 12 having a thickness of 1 mm and having a desired shape punched out in the center thereof and horizontally sent at a prescribed speed between a die 11 as disposed in the upper surface and a magnet 13 as disposed in the lower surface. The heat generating composition 2 of Example 5 is sent into a mold hole 12a from the upper surface of the mold 12 through a hole 11a of the die 11. The heat generating composition 2B is leveled in the same level as in the mold 12 by a leveling plate 15 as placed forward in the advancing direction and accommodated in the mold hole 12a, whereby a shape having a thickness of 1.5 mm is molded on the substrate 3. Thereafter, the mold 12 is removed to obtain a heat generating composition molded body as laminated on the substrate 3.

While not illustrated, a styrene-isoprene-styrene block copolymer (SIS) based sticky polymer is then provided in a network-like form on the surface of the heat generating composition molded body by the melt blow method, a covering material is covered thereon, and the periphery of the heat generating composition molded body is sealed by heat seal, followed by cutting into a desired shape. There is thus obtained a heat generating body having a desired shape. In addition, the cut heat generating body is subsequently sent into a packaging step and sealed in an air-tight outer bag. Furthermore, the same molding is possible even by changing the leveling plate by a pushing leveling plate.

Fig. 11 shows a leveling plate 15, and Fig. 12 shows a pushing leveling plate 15A.

Incidentally, so far as a pushing leveling function is kept, the tip of the pushing leveling plate may be rounded by trimming, namely, it may be deformed in any form by means of rounding, etc.

**Claims**

1. A process for producing a heat generating mixture, **characterized by** bringing a reaction mixture containing, as essential components, an iron powder, a reaction accelerator and water and having a water content of from 0.5 to 20 % by weight and a water mobility value showing an amount of surplus water of less than 0.01 with an oxidizing gas under circumstances at 0 °C or higher and regulating a temperature rise of the reaction mixture at 1 °C or higher within 10 minutes.

2. The process for producing a heat generating mixture according to claim 1, **characterized in that** an amount of the reaction accelerator is from 2 to 6 parts by weight based on 100 parts by weight of the total sum of the reaction accelerator and water.

3. The process for producing a heat generating mixture according to claim 1, **characterized in that** the reaction mixture is embedded in an air-permeable sheet-like material such as non-woven fabrics and subjected to a contact treatment with the oxidizing gas.

4. A heat generating mixture, **characterized by** being produced by the production process according to claim 1.

5. A heat generating composition, **characterized in that** the heat generating mixture according to claim 4 is used as a raw material; the water content is adjusted; an iron powder, a carbon component, a reaction accelerator and water are contained as essential components; and a water mobility value thereof is from 0.01 to 20.

6. The heat generating composition according to claim 5, **characterized in that** the heat generating composition contains at least one member selected from additional components consisting of a water retaining agent, a water absorptive polymer, a pH adjusting agent, a hydrogen formation inhibitor, an aggregate, a fibrous material, a functional substance, a surfactant, an organosilicon compound, a pyroelectric substance, a moisturizer, a fertilizer component, a hydrophobic polymer compound, a heat generating aid, a metal other than iron, a metal oxide other than iron oxide, an acidic substance, and a mixture thereof.

7. A heat generating body, **characterized in that** the heat generating composition according to claim 5 is accommodated in an air-permeable accommodating bag to form an exothermic part.

8. The heat generating body according to claim 7, **characterized in that** the accommodated heat generating composition is a heat generating composition molded body.

9. The heat generating body according to claim 7, **characterized in that** the air-permeable accommodating bag is constituted of a substrate and an air-permeable covering material, the heat generating composition is sectioned into plural parts, and the periphery of the heat generating composition is sealed to form plural sectional exothermic parts.

10. The heat generating body according to claim 8, **characterized in that** the shape of at least one member selected from the heat generating composition molded body, the sectional exothermic part and the exothermic part is a shape selected from the group consisting of a circular shape, an elliptical shape, a polygonal shape, a star shape, and a flower shape with respect to the planar shape and is a shape selected from the group consisting of a polygonal pyramidal shape, a conical shape, a frustum shape, a spherical shape, a parallelepiped shape, a cylindrical shape, a semi-pillar shape, a semicylindroid shape, a semicylidrical shape, a pillar shape, and a cylindroid shape with respect to the three-dimensional shape.

11. The heat generating body according to claim 7, **characterized in that** fixing means is provided in at least a part of the exposed surface of the heat generating body.

## FIG. 1

## FIG. 2

## FIG. 3

## FIG. 4

## FIG.5

## FIG.6

## FIG.7

## FIG.8

## FIG.9

## FIG. 10

## FIG. 11

## FIG. 12

# FIG.13

# FIG.14

## FIG.15

## FIG.16

## FIG.17

## INTERNATIONAL SEARCH REPORT

| | International application No. |
|---|---|
| | PCT/JP2005/012998 |

**A. CLASSIFICATION OF SUBJECT MATTER**
Int.Cl$^7$  A61F7/08, C09K5/16

According to International Patent Classification (IPC) or to both national classification and IPC

**B. FIELDS SEARCHED**

Minimum documentation searched (classification system followed by classification symbols)
Int.Cl$^7$  A61F7/08, C09K5/16

Documentation searched other than minimum documentation to the extent that such documents are included in the fields searched
Jitsuyo Shinan Koho          1922-1996   Jitsuyo Shinan Toroku Koho   1996-2005
Kokai Jitsuyo Shinan Koho   1971-2005   Toroku Jitsuyo Shinan Koho   1994-2005

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| A | JP 2002-155273 A  (Kaoru USUI),<br>28 May, 2002 (28.05.02),<br>Full text; Figs. 1 to 14<br>(Family: none) | 1-11 |
| A | JP 7-265347 A  (Yukio KOMATSU),<br>17 October, 1995 (17.10.95),<br>Full text; Figs. 1 to 10<br>(Family: none) | 1-11 |
| A | JP 2003-102761 A  (Kao Corp.),<br>08 April, 2003 (08.04.03),<br>Full text; Figs. 1 to 2<br>& US 2005/0000827 A1     & EP 1437111 A1<br>& WO 2003/028597 A1     & CN 1561187 A | 1-11 |

☒  Further documents are listed in the continuation of Box C.          ☐  See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 23 August, 2005 (23.08.05) | 13 September, 2005 (13.09.05) |

| Name and mailing address of the ISA/ | Authorized officer |
|---|---|
| Japanese Patent Office | |
| Facsimile No. | Telephone No. |

Form PCT/ISA/210 (second sheet) (January 2004)

40

**INTERNATIONAL SEARCH REPORT**

International application No.

PCT/JP2005/012998

C (Continuation).   DOCUMENTS CONSIDERED TO BE RELEVANT

| Category* | Citation of document, with indication, where appropriate, of the relevant passages | Relevant to claim No. |
|---|---|---|
| A | JP 1-201253 A  (Japan Pionics Co., Ltd.),<br>14 August, 1989 (14.08.89),<br>Full text; Figs. 1 to 6<br>(Family: none) | 1-11 |
| A | JP 57-200317 A  (Teijin Ltd.),<br>08 December, 1982 (08.12.82),<br>Full text<br>(Family: none) | 1-11 |
| A | JP 57-166156 A  (Dainihon Jochugiku Co., Ltd.),<br>13 October, 1982 (13.10.82),<br>Full text<br>(Family: none) | 1-11 |
| A | JP 56-28266 A  (Matsushita Electric Industrial Co., Ltd.),<br>19 March, 1981 (19.03.81),<br>Full text; Figs. 1 to 2<br>(Family: none) | 1-11 |

Form PCT/ISA/210 (continuation of second sheet) (January 2004)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 53060885 A
- JP 57010673 A
- JP 6343658 A
- JP 59189183 A
- JP 9075388 A
- JP 3161605 B
- JP 11508314 T
- JP 2002514104 T
- JP 2001507593 T
- JP 4293989 A

- JP 7194641 A
- JP 2002200108 A
- JP 10265373 A
- JP 9087173 A
- JP 6145050 A
- JP 6199660 A
- JP 10279466 A
- JP 10182408 A
- JP 11508786 T